# EUROPEAN PATENT APPLICATION

(11) **EP 3 640 329 A1**
(43) Date of publication of application: **22.04.2020**
(21) Application number: 18382746.8
(22) Date of filing: 18.10.2018
(51) Int. Cl.: C12N 9/22, A61K 38/46, A61K 48/00, C12N 15/10, C12N 15/11, C12N 15/113, C12N 15/62, A61P 35/00

(54) **GENE EDITING BASED CANCER TREATMENT**

(71) Applicant: Fundación del Sector Público Estatal Centro Nacional de Investigaciones Oncológicas Carlos III (F.S.P. CNIO), 28029 Madrid (ES)
(72) Inventor: RODRIGUEZ PERALES, Sandra, 28029 Madrid (ES); TORRES RUIZ, Raúl, 28029 Madrid (ES); MARTINEZ-LAGE, Marta, 28029 Madrid (ES)
(74) Representative: Elzaburu S.L.P.

(57) **Abstract**

The present invention relates to a method for eliminating cancer cells, wherein said cells comprise a genomic rearrangement which leads either to the expression of a fusion gene not present in non-cancer cells, or to genomic amplifications or rearrangements which lead to the induction of the expression or to the overexpression of a cancer inducing gene, said method comprising: (a) cleaving the genome in at least two sites, said cleavage leading to either a deletion, an inversion, a frameshift and/or an insertion in the genome of said cancer cells, and/or (b) cleaving the expression product of said fusion gene or cancer inducing gene in at least one site.

## Description

### FIELD OF THE INVENTION

The present invention belongs to the field of Biomedicine and relates to a gene-editing based cancer treatment where cancer cells are selectively eliminated.

### BACKGROUND OF THE INVENTION

Specific, recurrent chromosomal rearrangements are very common and well-known hallmarks of cancer. Genes affected by chromosome aberrations, in particular translocations, deletions and inversions, fall into two categories: proto-oncogenes that undergo enforced expression as a result of their new chromosomal context, or fusion genes where the breakpoints are within introns of the affected genes on the two involved chromosomes. The latter is the more common consequence of chromosomal translocations, and results in the creation of new chimeric genes consequence of the fusion of the coding sequences of two different genes¹. The introduction of next-generation sequencing (NGS) technologies has dramatically changed the gene fusion landscape providing a radically new means to identify fusions. Using NGS, a plethora of gene fusions (more than 9,000) has now been identified². To date, more than 350 recurrent fusion genes involving more than 300 different genes have been identified¹⁰. Although the products of oncogenic fusion genes are diverse, they can primarily be classified into two groups, transcription factors and tyrosine kinases (TKs).

Fusion genes have critical functions in tumorigenesis and are exceptionally powerful cancer mutations, as they often have multiple effects on a target gene: in a single 'mutation' they can dramatically change expression, remove regulatory domains, force oligomerization, change the subcellular location of a protein or join it to novel binding domains. This is reflected clinically in the fact that some neoplasms are classified or managed according to the presence of a particular fusion gene³. Fusion genes are tumour-specific and therefore important targets for therapy: promyelocytic leukaemias that have PML-RARα fusion of the retinoic acid receptor-α are treated with retinoic acid⁴, and the BCR- ABL fusion gene of chronic myeloid leukaemia is the target of the iconic targeted drug Glivec (STI-571)⁵.

There is strong evidence that gene fusions represent important and early steps in the initiation of carcinogenesis. First, they are usually closely correlated with specific tumour phenotypes⁶⁻¹⁰. Second, it has been shown that successful treatment is paralleled by a decrease or eradication of the disease-associated chimera¹¹⁻¹⁴. And finally, silencing fusion transcripts in vitro leads to the reversal of tumorigenicity, decreased proliferation and/or differentiation^{15,16}.

### Gene fusion products as tumour specific therapeutic targets

Gene fusions produce tumour-specific molecules because the chimeric RNA and protein product only occurs in the cell with the chromosomal rearrangement (translocation, deletion or inversion). These unique molecules are potential tumour specific therapeutic targets. One important problem of gene fusion products as therapeutic targets is their intracellular location. Although intracellular delivery of therapeutic molecules is challenging, their tumour specificity is an important motivating factor for developing new-targeted therapies. The flow of genetic information from DNA to mRNA and to proteins has several points at which therapeutic reagents could intervene. Several approaches have been developed to target gene fusions, including:
1.- Targeting protein: small molecules, intrabodies and aptamers.
2.- Targeting mRNA: antisense, ribozymes and RNAi.
3.- Targeting DNA: genome editing: The CRISPR-Cas9 systems, which can generate targeted breaks in the genome at any desired location allowing direct gene editing, can be used to target chromosomal DNA breakpoints that create the fusion genes providing a genotype-specific approach to treating human cancers¹⁷. The Cas9 is a DNA endonuclease that can be targeted to a specific 20-bp DNA sequence by a single guide RNA (sgRNA)^{18,19}. Luo's group²⁰, by using Cas9 nickase mediated genome editing, were able to insert HSV1-tk into patient specific chromosomal breakpoints of the fusion genes TMEM-CCDC67 and MAN2A1-FER, found in prostate cancer and hepatocellular carcinoma, respectively. Treatment of tumours bearing these chromosome breakpoints with ganciclovir after induction of HSV1-tk led to cell death in cell culture and to a decrease in tumour size and mortality in mice xenografted with human prostates and liver cancers. Although genotype-specific, this therapy approach relies on a knock-in strategy that nowadays is associated with low efficiencies (0.1-10 %). On the other hand, this approach depends on the previous knowledge of the breakpoint sequence which is patient specific what makes necessary a sequencing study of the introns more probably involved in the translocation together with the design and development of new targeting tools (sgRNAs and donor template) for the treatment of each particular patient. This approach could also be associated with wild type cell death events associated with TK random integration.

Working in the same direction of targeting cancer fusion genes that do not exist in normal cells but using a more efficient and no patient specific strategy, the inventors have developed a radically simple, versatile, highly efficient and clinically relevant gene editing approach based on the targeted deletion of a large genomic region containing the fusion oncogene leaving unaltered the exonic regions of their corresponding wild-type alleles. The CRISPR-Cas9 approach is based on an efficient (30-80 %) knock-out strategy to selectively destroy cancer cells that harbour recurrent fusion genes whilst sparing the normal counterparts.

WO2016094888 A1 relates to the use of CRISPR and compositions comprising a guide RNA and a Cas protein, specifically for introducing a suicidal gene into in the breakpoint loci of a cancer-specific target sequence which is a fusion gene.

There is still a need of therapeutic tools against cancer which are universal and not patient specific, and which are more efficient and act only on cancer cells, minimizing the side effects of the treatment.

### DESCRIPTION OF THE INVENTION

The present invention provides a way to eliminate cancer cells specifically using endonuclaease(s) that cleave the genome at specific sites, which results in a selective elimination of the cancer inducing gene and thereby elimination of the cancer cells.

The cleavage is directed to the genomic rearrangement which leads either to the expression of a fusion gene absent in non-cancer cells, or to genomic amplifications or rearrangements which lead to the induction of the expression or to the overexpression of a cancer inducing gene. The inventors have found a simple and straightforward way to design a treatment which is universal (not patient specific), since it does not depend on the specific sequence (breakpoint) where the genomic rearrangement is occurring. For those cancers where there is a fusion gene and fusion protein, the present invention allows the truncation or the elimination of the fusion protein, which in turn leads to the death of the cancer cell. But more importantly, the present invention provides a therapy with minimal side effects since the modification of the coding regions of the genome will only take place in cells carrying the genomic rearrangement, i.e. in cancer cells.

Thus, in a first aspect, the present invention relates to a method for eliminating cancer cells, wherein said cells comprise a genomic rearrangement which leads either to the expression a fusion gene not present in non-cancer cells, or to genomic amplifications or rearrangements which lead to the induction of the expression or to the overexpression of a cancer inducing gene, said method comprising: (a) cleaving the genome in at least two sites, said cleavage leading to either a deletion, an inversion, a frameshift and/or an insertion in the genome of said cancer cells, or (b) cleaving the expression product of said fusion gene or cancer inducing gene in at least one site.

In a preferred embodiment, the method comprises cleaving in at least two, three, four or five sites or even further cleavage sites, for example in the case of gene amplifications, the cleavage site may occur in as many sites as repetitions of the amplified gene are present. Therefore, the method may comprise cleaving in at least two sites to hundreds of sites in cases where the genomic rearrangement comprises hundreds of repetitions of a cancer inducing gene. In a preferred embodiment, the method comprises successive repetitions of the cleavage targeting the same or different cleaving sites. In a preferred embodiment, the method comprises cleaving in two sites and subsequently cleaving in two sites that may be the same or different. For this successive cleavage, nested gRNAs may be employed.

As used herein, the term "cancer inducing gene" refers to an oncogene or a proto-oncogene that has the potential to cause cancer.

In a preferred embodiment, the cleavage is performed by at least one endonuclease. Said endonuclease may be a CRISPR related protein such as Cas9 or by a functional equivalent thereof, whose target site is driven by the sequence of the guide RNA. Also, the cleaving may be performed by endonucleases such as a zinc-finger nucleases (ZFN) or transcription activator-like effector nucleases (TALEN). In these cases, the target site is inherent to the nuclease and therefore at least two nucleases will be necessary to cleave the genome in at least two sites. Both of these approaches involve applying the principles of protein-DNA interactions of these domains to engineer new proteins with unique DNA-binding specificity. These methods have been widely successful for many applications.

In a preferred embodiment of the method of the first aspect, the cancer cells comprise a genomic rearrangement which leads to the expression of the rearranged gene not present in non-cancer cells. Preferably, it leads to the expression of a fusion gene not present in non-cancer cells.

In a preferred embodiment of the method of the first aspect, said method comprises cleaving the genome in two sites, or in three sites or in four sites. Preferably, it consists in cleaving the genome in two sites. Preferably, it consists in cleaving the genome in three sites. Preferably, it consists in cleaving the genome in four sites.

In a preferred embodiment of the method of the first aspect, cleaving the genome leads to a deletion, an inversion, a frameshift or any combination thereof.

In a preferred embodiment of the method of the first aspect, at least two of the cleaving sites are in introns.

The inventors have observed that upon cleaving the genome of the cancer cells in two sites, it can occur that the cleaved sequence inserts itself in the same position but in inverse orientation (an inversion), which leads to the death of the cancer cell because either the fusion protein is not produced (the inversion leads to a truncated protein) or the induction of the expression or to the overexpression of a cancer inducing gene is prevented.

The expression "genomic rearrangement" refers to a deletion, an insertion or a genomic amplification. Also, a genomic rearrangement may be a translocation of a chromosomal region, such as those that lead to the production of fusion proteins.

Preferred genomic rearrangements are listed in the following table 1.

| | **Disease** | **Fusion Gene** |
|---|---|---|
| LEUKEMIA | Acute myeloid leukemia (AML) | *RUNX1-RUNX1T1* |
| | | *CBFB-MYH11* |
| | | *KMT2A-MLLT3* |
| | | *RPN1-MECOM* |
| | | *DEK-NUP214* |
| | | *PVT1-MECOM* |
| | | *RUNX1-MECOM* |
| | Acute promyelocytic leukemia (APL) | *PML-RARA* |
| | | *ZBTB16-RARA* |
| | Acute lymphocytic leukemia (ALL) | *ETV6-RUNX1* |
| | | *BCR-ABL1* |
| | | *TCF3-PBX1* |
| | | *KMT2A-AFF1* |
| | | *PICALM-MLLT10* |
| | | *IGH-CEBPA* |
| | | *TCF3-HLF* |
| | | *TRA-MYC* |
| | Chronic myeloid leukemia (CML) | *BCR-ABL1* |
| | Chronic lymphocytic leukemia (CLL) | *IGH-BCL1* |
| | | *IGH-BCL2* |
| | | *IGH-BCL3* |
| SARCOMA/BONE | Ewing's sarcoma | *EWS-FLI1* |
| | | *EWS-ERG* |
| | | *EWS-ETV1* |
| | | *EWS-FEV* |
| | | *EWS-E1AF* |
| | Alveolar rhabdomyosarcoma | *PAX3*/*FOXO1* |
| | | *PAX7-FOXO1* |
| | Congenital spindle cell RMS | *VGLL2-CITED2* |
| | | *VGLL2-NCOA2* |
| | | *TEAD1-NCOA2* |
| | Alveolar soft-part sarcoma | *ASPSCR1-TFE* |
| | Extraskeletal myxoid chondrosarcoma | *EWS-TEC* |
| | | *TAF2N-TEC* |
| | Fibromyxoid sarcoma | *FUS-CREB312* |
| | Endometrial stromal sarcoma | *JAZF1-JJAZ1* |
| | Angiomatoid fibrous histiocytoma | *EWSR1-CREB1* |
| | | *FUS-ATF1* |
| | Juvenile fibrosarcoma | *ETV6-NTRK3* |
| | Myxoid chondrosarcoma | *EWS-NR4A3* |
| | | *TFC12-NR4A3* |
| | | *TAF2N-NR4A3* |
| | Synovial sarcoma | *SYT-SSX1* |
| | | *SYT-SSX2* |
| | | *SYT-SSX4* |
| | Mixoid liposarcoma | *FUS-CHOP* |
| | | *EWS-CHOP* |
| | Spindle cell sarcoma | *MLL4-GPS2* |
| | Dermatofibrosarcoma protuberans (DFSP) | *COL1A1-PDGFB* |
| | Clear cell sarcoma | *EWS-ATF1* |
| | Soft tissue angiofibroma | *AHRR-NCOA2* |
| | URCS | *BCOR-CCNB3* |
| | | *CIC-DUX4L10* |
| | | *CIC-DUX4* |
| | Chondroid lipoma | *C11ORF95-MKL2* |
| | Mesenchymal chondrosarcoma | *HEY1-NCOA2* |
| | Biphenotypic sinonasal sarcoma | *PAX3-MAML3* |
| | Despoplastic small round cell tumor | *EWS-WT1* |
| LYMPHOMAS | Follicular lymphoma | *BCL2-IGH* |
| | Mantle lymphoma | *BCLI-IGH* |
| | large cell lymphoma | *NPM-ALK* |
| | Burkit lymphoma | *cMYC-IGH* |
| BRAIN TUMORS | Pilocytic astrocytoma | *KTAA1549-BRAF* |
| | Glioblastoma | *TPM3-NTRK1* |
| | | *FGFR3-TACC3* |
| | sporadic pilocytic astrocytomas/some | *KIAA1549-BRA* |
| | pedriatic brain tumors | |
| | supratentorial ependymomas | *C11orf95-RELA* |
| | Meningioma | *MN1-ETV6* |
| LIVER TUMORS | fibrolamellar hepatocellular carcinoma | *DNAJB1_PRKACA* |
| KIDNEY TUMORS | Clear renal cell carcinoma | *SFPQ-TFE3* |
| | | *TFG-GPR1228* |
| | Mesoblastic nephroma | *ETV6-NTRK3* |
| | Renal cell carcinoma | *MALA T1-TFEB* |
| LUNG TUMORS | Lung adenocarcinoma | *EML4-ALK* |
| | | *LRIG3*/*ROS1* |
| | Non-small cell carcinoma | *EML4*/*ALF* |
| PROSTATE TUMORS | Prostate | *TMPRSS2-ERG* |
| BREAST/OVARIAN TUMORS | Breast Cancer | *BCAS4-BCAS3* |
| | | *TEL1XR1-RGS17* |
| | | *ODZ4-NRG1* |
| | Secretory breast cancer | *ETV6-NTRK3* |
| | Serous ovarian carcinoma | *ESRRA-C11orf20* |
| COLON TUMORS | Colorectal Cancer | *PTPRK-RSPO3* |
| | | *TPM3-NTRK1* |
| | | *EIF3E-RSPO2* |
| BLADDER TUMORS | Bladder cancer | *FGFR3-TACC3* |
| SALIVARY GLAND TUMORS | Mucoepidermoid carcinomas | *MECT1-MAML2* |
| | Adenoid cystic carcinoma | *MYC-NFIB* |
| | Pleomorphic adenoma | *CTNNB1-PLAG1* |
| ENDOCRINE CANCER | Papillary thyroid cancer (PTC) | *ETV6-NTRK3* |
| | follicular thyroid cancer | *PAX8-PPARG* |
| OTHER CANCER | Aggressive midline carcinoma | *BRD4-NUT* |
| | Melanoma of soft parts | *EWSRI-ATF1* |
| | Gastric cancer | *CD33-SLC1A2* |

More preferred genomic rearrangements are fibrolamellar hepatocellular carcinoma, non-small cell lung cancer, alveolar rhabdomyosarcoma, glioblastoma, colorectal cancer, acute lymphocytic leukemia, Ewing sarcoma and bladder cancer.

Insertions may vary in size, from a few nucleotides to hundreds or more than a thousand nucleotides. Said insertions can include codifying sequences or non codifying sequences. They can also include a suicide gene, which is inserted after cleaving the genome in at least two sites. The inserted DNA can either be endogenous or exogenous.

In a preferred embodiment, the genomic rearrangement is other than an insertion. In a preferred embodiment, the genomic rearrangement is an insertion of a sequence other than a suicide gene.

In a preferred embodiment of the first aspect, at least two of the cleaving sites are in introns chosen in a way that the mature mRNA resulting from the fusion gene after the deletion is truncated or has a different sequence due to a frameshift. In the case of translocations that bind one promoter to the coding sequence of another gene, one of the sgRNAs will have its target domain in an intron and the other will have its cleavage site located before or after the promoter sequence but without affecting said sequence, so that the expression of the wild type gene controlled by this promoter is not altered.

The cancer cells may comprise both a genomic rearrangement that leads to a fusion gene and a genomic rearrangement that leads to the induction of the expression or to the overexpression of a cancer inducing gene, such as an oncogene. Also, the cancer cells may comprise both a genomic rearrangement that leads to a fusion gene and a genomic amplification that leads to the induction of the expression or to the overexpression of a cancer inducing gene, such as an oncogene.

The method of the invention achieves a cleavage of the genome in those at least two sites exclusively in the cancer cells because in the case of fusion genes, only the cancer cells have fusion genes, and in the case of genomic rearrangements leading to the induction of the expression or to the overexpression of a cancer inducing gene, only those cells have said genomic rearrangements. In the case of amplifications, the target domain of the gRNA (therefore the cleavage sequence) is repeated so there are at least two cleavage sites although only one gRNA is used, because the target domain is the same. The number of cleavage sites in case of amplifications will depend on the number of repetitions but only one single gRNA is necessary. Therefore, in this case the cleavage in at least two sites does not imply that the sites have different target domain sequences.

In a preferred embodiment of the first aspect, the cleavage does not result in the insertion of an exogenous gene, like a suicide gene, like the ones disclosed in WO2016094888 A1.

The term "fusion gene" as used herein means the codifying region of a gene and also, the regulatory regions and other non codifying sequences such as promoters, etc.

Another aspect of the present invention relates to a method for eliminating cancer cells, wherein said cells comprise a genomic rearrangement which leads the expression a fusion gene not present in non-cancer cells, said method comprising cleaving the expression product of said fusion gene in at least one site.

The cleavage of the mRNA of the fusion gene is specific for the cancer cells and leads to the degradation of the mRNA, preventing the translation of the fusion protein, which in turn leads to the death of the cancer cell.

In a preferred embodiment of this aspect, the cleavage is done using endonuclease Cas13. The cleavage of Cas13 of the RNA of the fusion gene is exclusive of the cancer cells and leads to the degradation of the RNA in the cell and eventually to its death. The Cas13 enzyme is a CRISPR RNA (crRNA)-guided RNA-targeting CRISPR effector²¹⁻²⁷. Under the guidance of a single crRNA, Cas13 can bind and cleave a target RNA carrying a complementary sequence. Through this mechanism, the CRISPR-Cas13 system can effectively knockdown mRNA expression in mammalian cells with an efficacy comparable with RNA interference technology and with improved specificity^{28,29}. X. Zhao and collaborators³⁰ have demonstrated that the CRISPR-Cas13 system can be engineered for the efficient and specific knockdown of mutant KRAS-G12D mRNA in pancreatic cancer models.

In a preferred embodiment of this aspect, only one gRNA is used. This gRNA has its targeting domain in the expression product of the fusion gene. Preferred gRNAs are those codified by sequences SEQ ID NO: 135, SEQ ID NO: 136, SEQ ID NO: 137 and SEQ ID NO: 138, useful for cleaving the expression products of fusion genes *DNAJBI-PRKACA, EML4-ALK, PAX3-FOXO1* and *TPM3-NTRK1,* respectively.

Another aspect relates to a kit of parts comprising an endonuclease capable of cleaving a messenger RNA (mRNA), such as the CRISPR associated protein Cas13 or another endonuclease derived from said Cas13 or a functional equivalent thereof; and at least one gRNA, preferably one gRNA, with its targeting domain in the expression product of a fusion gene present in cancer cells and absent in non-cancer cells. In a preferred embodiment, the kit comprises the nuclease of SEQ ID NO: 126 or a functional equivalent thereof and a gRNA selected from SEQ ID NO: 144, SEQ ID NO: 145, SEQ ID NO: 146 and SEQ ID NO: 147. In a preferred embodiment, the kit consists essentially of the nuclease of SEQ ID NO: 126 or a functional equivalent thereof and a gRNA selected from SEQ ID NO: 144, SEQ ID NO: 145, SEQ ID NO: 146 and SEQ ID NO: 147. Preferably, the sequence that codifies for the nuclease of SEQ ID NO: 126 is SEQ ID NO: 127.

Another aspect relates to a nucleic acid codifying for a nuclease capable of cleaving a messenger RNA (mRNA), such as the CRISPR associated protein Cas13 or another endonuclease derived from said Cas13 or a functional equivalent thereof; and at least one gRNA, preferably one gRNA, with its targeting domain in the expression product of a fusion gene present in cancer cells and absent in non-cancer cells. In a preferred embodiment, the nucleic acid codifies for the nuclease of SEQ ID NO: 126 or a functional equivalent thereof and for a gRNA selected from SEQ ID NO: 144, SEQ ID NO: 145, SEQ ID NO: 146 and SEQ ID NO: 147.

Another aspect relates to the use of the above mentioned kits or nucleic acids for the treatment of cancer, preferably for the treatment of fibrolamellar hepatocellular carcinoma, non-small cell lung cancer, alveolar rhabdomyosarcoma, colorectal cancer, acute lymphocytic leukemia and Ewing sarcoma.

In a preferred embodiment of the method of the first aspect, the cleaving is done by an endonuclease selected from a CRISPR associated protein, a zinc-finger nuclease (ZFN) and a transcription activator-like effector nuclease (TALEN). Preferably, the cleaving is done by a Cas protein, preferably Cas9 or a functional equivalent thereof. In a preferred embodiment, the target of said endonuclease is in an exon or intron of a fusion gene present in cancer cells and absent in non-cancer cells and wherein said target is not patient-specific. The target of said endonuclease may be in an intron or an exon or a non coding sequence including promoter and 5' and 3' ends.

In a preferred embodiment of the method of the first aspect, at least two guide RNAs are used to target the cleaving of the genome.

A second aspect of the present invention related to a kit of parts comprising at least two endonucleases, preferably selected from a zinc-finger nuclease (ZFN) and a transcription activator-like effector nuclease (TALEN), wherein said endonucleases specifically cleave the genome in at least two sites (each endonuclease cleaves in one specific site) and wherein said cleavages lead to either a deletion, a frameshift and/or an insertion in the genome, preferably a deletion and/or a frameshift.

In a preferred embodiment, the kit of parts comprises at least two ZFNs or a nucleic acid encoding at least two ZFNs. In another preferred embodiment, the kit of parts comprises at least two TALENs or a nucleic acid encoding at least two TALENs. In another preferred embodiment, the kit of parts comprises at least one ZFN and at least one TALEN or a nucleic acid encoding at least one ZFN and at least one TALEN.

In a preferred embodiment, the kit of parts comprises: (a) a CRISPR associated endonuclease, preferably a Cas protein, more preferably Cas9 or Cas13, even more preferably Cas9 or a functional equivalent thereof; and (b) at least one gRNA is used to target the cleaving of the genome, preferably at least two gRNAs are used to target the cleaving of the genome. In a preferred embodiment, the kit of parts comprises (a) a CRISPR associated endonuclease, preferably a Cas protein, more preferably Cas9 or Cas13 or a functional equivalent thereof, even more preferably Cas9; and (b) at least a pair of gRNAs that have a targeting domain in a genomic rearrangement present in a cancer cell which leads either to the expression a fusion gene not present in non-cancer cells, or to genomic amplifications or rearrangements which lead to the induction of the expression or to the overexpression of a cancer inducing gene. In a preferred embodiment, the kit of parts consists of a CRISPR associated endonuclease, preferably a Cas protein, more preferably Cas9 or Cas13 or a functional equivalent thereof, even more preferably Cas9; and at least one gRNA, preferably a pair of gRNAs that have a targeting domain in a genomic rearrangement present in a cancer cell which leads either to the expression a fusion gene not present in non-cancer cells, or to genomic amplifications or rearrangements which lead to the induction of the expression or to the overexpression of a cancer inducing gene. As used herein, the term "guide RNA" and "single guide RNA" are used interchangeably and are abbreviated as "gRNA" and "sgRNA".

A preferred embodiment of the kit of parts of the present invention comprises: (a) the nuclease with amino acid sequence SEQ ID NO: 1; and (b) the pair of gRNAs with nucleotide sequences SEQ ID NO: 2 and SEQ ID NO: 3; or the pair of gRNAs with nucleotide sequences SEQ ID NO: 4 and SEQ ID NO: 5; or a pair of gRNAs with nucleotide sequences SEQ ID NO: 128 or SEQ ID NO: 129 and SEQ ID NO: 130 or SEQ ID NO: 131; or a pair of gRNAs with nucleotide sequences SEQ ID NO: 132 or SEQ ID NO: 133 and SEQ ID NO: 134 or SEQ ID NO: 135; or a pair of gRNAs with nucleotide sequences SEQ ID NO: 136 or SEQ ID NO: 137 and SEQ ID NO: 138 or SEQ ID NO: 139; or a pair of gRNAs with nucleotide sequences SEQ ID NO: 140 or SEQ ID NO: 141 and SEQ ID NO: 142 or SEQ ID NO: 143.

Another aspect of the present invention relates to a nucleic acid comprising the codifying sequence for (a) a CRISPR associated endonuclease, preferably a Cas protein, more preferably Cas9 or Cas13 or a functional equivalent thereof, even more preferably Cas9; and (b) at least one gRNA that has a targeting domain in the expression product of a fusion gene or at least a pair of gRNAs that have a targeting domain in a genomic rearrangement present in a cancer cell which leads either to the expression of a fusion gene not present in non-cancer cells, or to genomic amplifications or rearrangements which lead to the induction of the expression or to the overexpression of a cancer inducing gene.

Another aspect of the present invention relates to a nucleic acid comprising essentially the codifying sequence for (a) a CRISPR associated endonuclease, preferably a Cas protein, more preferably Cas9 or Cas13 or a functional equivalent thereof, even more preferably Cas9; and (b) at least one gRNA that has a targeting domain in the expression product of a fusion gene or at least a pair of gRNAs that have a targeting domain in a genomic rearrangement present in a cancer cell which leads either to the expression of a fusion gene not present in non-cancer cells, or to genomic amplifications or rearrangements which lead to the induction of the expression or to the overexpression of a cancer inducing gene. Said nucleic acid may comprise other elements, such as promoters, enhancers, etc. well known to the skilled person and which allow the expression of the endonuclesase and the gRNAs in the target cancer cells.

A particularly preferred embodiment of the present invention relates to a nucleic acid comprising the codifying sequence for: (a) the nuclease with amino acid sequence SEQ ID NO: 1, preferably the codifying sequence SEQ ID NO: 32, and (b) the pair of gRNAs with nucleotide sequences SEQ ID NO: 2 and SEQ ID NO: 3; or the pair of gRNAs with nucleotide sequences SEQ ID NO: 4 and SEQ ID NO: 5; or a pair of gRNAs with nucleotide sequences SEQ ID NO: 128 or SEQ ID NO: 129 and SEQ ID NO: 130 or SEQ ID NO: 131; or a pair of gRNAs with nucleotide sequences SEQ ID NO: 132 or SEQ ID NO: 133 and SEQ ID NO: 134 or SEQ ID NO: 135; or a pair of gRNAs with nucleotide sequences SEQ ID NO: 136 or SEQ ID NO: 137 and SEQ ID NO: 138 or SEQ ID NO: 139; or a pair of gRNAs with nucleotide sequences SEQ ID NO: 140 or SEQ ID NO: 141 and SEQ ID NO: 142 or SEQ ID NO: 143.

Preferred pairs of gRNAs are listed below for four preferred cancers (two gRNAs are provided for each cleavage site for each disease):

### Fibrolamellar hepatocellular carcinoma.

### Fusion gene DNAJB1-PRKACA

### > DNAJB1

**SEQ ID NO: 128:**
   **Position** 11252; **Strand:** 1; **Sequence:** GATGTCGCGTGTCGCTGAAA; **PAM:** GGG;
   **Specificity Score:** 98.2633296; **Efficiency Score:** 46.297448948149196
**SEQ ID NO: 129:**
   **Position** 12068; **Strand:** 1; **Sequence:** CAGGAGCCGACCCCGTTCGT; **PAM:** GGG;
   **Specificity Score:** 95.6957217; **Efficiency Score:** 54.78256070394193

### > PRKACA

**SEQ ID NO: 130:**
   **Position:** 1935; **Strand:** 1; **Sequence:** GTCGGAACTATTGGTCGAAA; **PAM:** AGG;
   **Specificity Score:** 94.8121995; **Efficiency Score:** 49.440679428197285
**SEQ ID NO: 131:**
   **Position:** 846; **Strand:** 1; **Sequence:** CATGGCACGTATGACCGCTG; **PAM:** GGG;
   **Specificity Score:** 91.353957; **Efficiency Score:** 69.65437430185875

### Non-small cell lung cancer. Fusion gene EML4-ALK

### > EML4

**SEQ ID NO: 132:**
   **Position:** 42262641; **Strand:** 1; **Sequence:** ACTTATAAGTATAGGGAATC; **PAM:** AGG;
   **Specificity Score:** 73.9472732; **Efficiency Score:** 41.01080196055957
**SEQ ID NO: 133:**
   **Position:** 42263040; **Strand:** -1; **Sequence** GGATTAGTTGAAAGACTGCC:; **PAM:** TGG;
   **Specificity Score:** 71.6133454; **Efficiency Score:** 42.03495303486019

### > ALK

**SEQ ID NO: 134:**
   **Position:** 698882; **Strand:** 1; **Sequence:** GTCCACTAAATGTGACGCCC; **PAM:** AGG;
   **Specificity Score:** 92.5531067; **Efficiency Score:** 54.886608266105895
**SEQ ID NO: 135:**
   **Position:** 698375; **Strand:** 1; **Sequence:** GAGGACAAGCCTTGACATTC; **PAM:** AGG;
   **Specificity Score:** 73.6854929; **Efficiency Score:** 31.53386778402246

### Alveolar Rhabdomyosarcoma. Fusion gene PAX3-FOXO1

### > PAX3

**SEQ ID NO: 136:**
   **Position:** 96631; **Strand:** 1; **Sequence:** TGCAGTCAGATGTTATCGTC; **PAM:** GGG;
   **Specificity Score:** 92.4221555; **Efficiency Score:** 51.18110845580498
**SEQ ID NO: 137:**
   **Position:** 95396; **Strand:** 1; **Sequence:** TACTGGAACTCCTAGATCCG; **PAM:** AGG;
   **Specificity Score:** 87.0387327; **Efficiency Score:** 65.91762085633988

### > FOXO1

**SEQ ID NO: 138:**
   **Position:** 108815; **Strand:** 1; **Sequence:** CAATGGTCCTTTGTCAAACG; **PAM:** AGG;
   **Specificity Score:** 83.6621655; **Efficiency Score:** 62.541738049196596
**SEQ ID NO: 139:**
   **Position:** 108095; **Strand:** -1; **Sequence:** TGGCAACGTGAACAGGTCCA; **PAM:** AGG;
   **Specificity Score:** 76.5677517; **Efficiency Score:** 64.81592708499454

### Glioblastoma. Fusion gene TPM3-NTRK1

### > TPM3

**SEQ ID NO: 140:**
   **Position:** 23359; **Strand:** -1; **Sequence:** AACCTGAATACATGGTAAGG; **PAM:** AGG;
   **Specificity Score:** 71.2689516; **Efficiency Score:** 62.792710664503396
**SEQ ID NO: 141:**
   **Position:** 23653; **Strand:** 1; **Sequence:** TACTCTTGCTCATCAAGCAG; **PAM:** GGG;
   **Specificity Score:** 69.2115539; **Efficiency Score:** 60.98480800289473

### > NTRK1

**SEQ ID NO: 142:**
   **Position:** 156877732; **Strand:** 1; **Sequence:** CTGGATGAGCAAGCGCTGTA; **PAM:** TGG;
   **Specificity Score:** 90.0534004; **Efficiency Score:** 47.93006273145852
**SEQ ID NO: 143:**
   **Position:** 156877536; **Strand:** -1; **Sequence:** TCAGAGAAGGACTAGACCGA; **PAM:**
   GGG; **Specificity Score:** 86.3585081; **Efficiency Score:** 68.2983905554927

Another aspect of the present invention relates to the use of any one of the kits of parts of the invention or the nucleic acids of the invention for the treatment of cancer, preferably for the treatment of fibrolamellar hepatocellular carcinoma, non-small cell lung cancer, alveolar rhabdomyosarcoma, glioblastoma, colorectal cancer, acute lymphocytic leukemia, Ewing sarcoma and bladder cancer. Preferably, for the treatment of cancers where there is a genomic rearrangement present in a cancer cell which leads either to the expression a fusion gene not present in non-cancer cells, or to genomic amplifications or rearrangements which lead to the induction of the expression or to the overexpression of a cancer inducing gene. More preferably, for the treatment of cancers where there is a fusion gene and a fusion protein specifically in cancer cells, not present in non-cancer cells. Even more preferably, for the treatment of the cancers listed in table 1. Preferably, the kit of parts of the present invention is delivered to the patient in need of the treatment by specific delivery systems that are known to be useful in each particular cancer type. Delivery systems such as viral vectors, adenoviral vectors, lentiviral vectors, AAVs and other delivery systems such as nanoparticles and macrocomplexes can be used. The administration of the kit of parts of the present invention can be through different ways, depending on the target tissue or cancer cell in the patient. Thus, the administration may be oral or parenteral, subcutaneous, intramuscular or intravenous, as well as intrathecal, intracranial, etc., depending on the patient needs.

### DESCRIPTION OF THE DRAWINGS

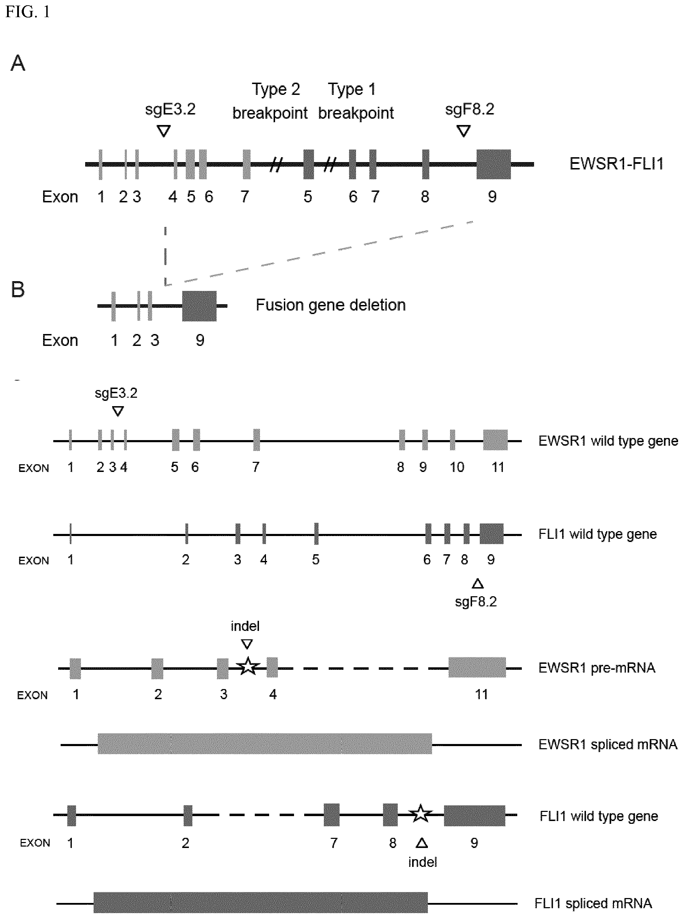

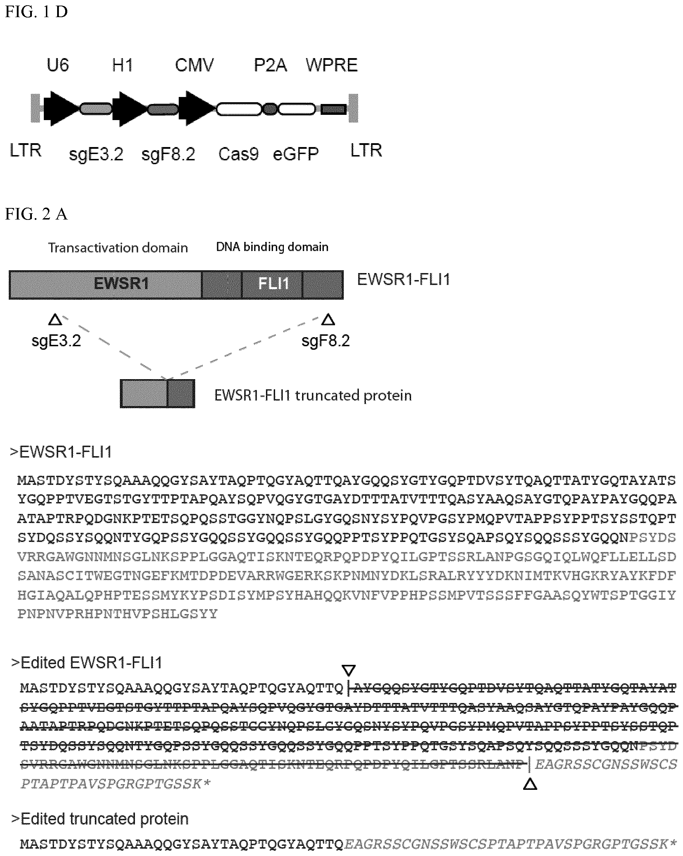

Figure 1: a. Schematic representation of the type 1 (*EWSR1* exon 7 fused to *FLI1* exon 6) and type 2 (*EWSR1* exon 7 fused to *FLI1* exon 5) *EWSR1-FLI1* fusion gene loci. Indicated are the sgRNAs targeting introns 3 of EWSR1 and 8 of FLI1 genes used to edit the fusion gene. b. Schematic representation of the truncated DNA generated by Cas9 edition. c. Gene editing effect on the *EWSR1* and *FLI1* WT intronic on-target sites: a. Schematic representation of *EWSR1* and *FLI1* WT genes. Indicated are the sgRNAs targeting both genes. d. Schematic representation of the indels removal by the splicing machinery. Schematic illustration of the pLVX-U6E3.2-H1F8.2-Cas9-2A-eGFP vector.
Figure 2: a. EWSR1-FLI1 chimeric protein and truncated protein generated by genome editing. b Amino acid sequence of the EWSR1-FLI1 protein (Type 1). Residues corresponding to EWSR1 or to FLI1 are shown in black or grey, respectively. c. Aminoacid sequence of the edited EWSR1-FLI1 truncated protein. Deleted residues are shown crossed out, the new residues generated by the change of reading frame after the mutation are double underlined. The premature STOP codon is shown with an asterisk.

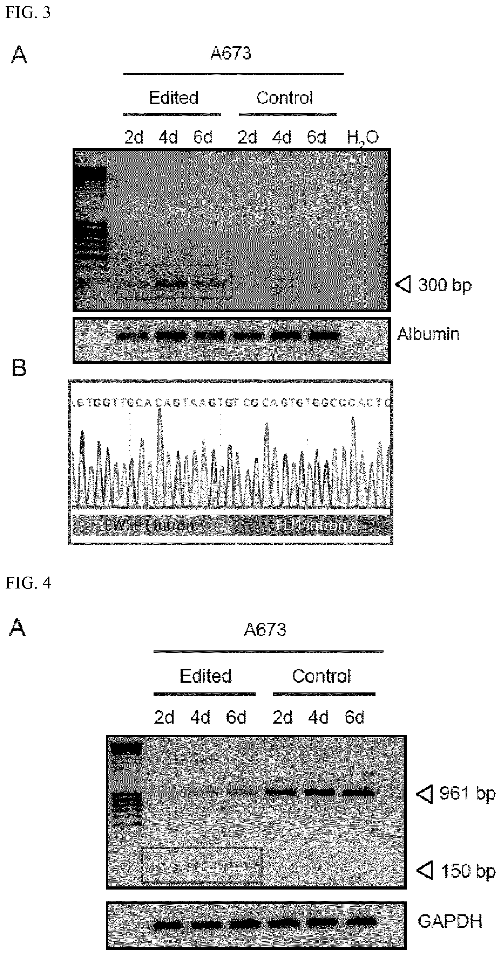

Figure 3. Analysis of *EWSRI-FLI1* DNA a. Agarose gel electrophoresis showing the results of genomic PCR analysis of edited and control A673 ES cell line using oligos flanking the DNA loci targeted by sgE3.2 and sgF8.2. The 300bp PCR fragment denote deletion of the DNA fragment between the loci targeted by sgEW3.2 and sgFLI8. PCR analysis was done using DNA from cell cultures on days 2, 4 and 6 post-transduction (pt). Albumin is used as an internal control of the PCR reaction. b. Sanger sequencing analysis of the PCR bands. A representative deleted sequence and chromatogram are shown.

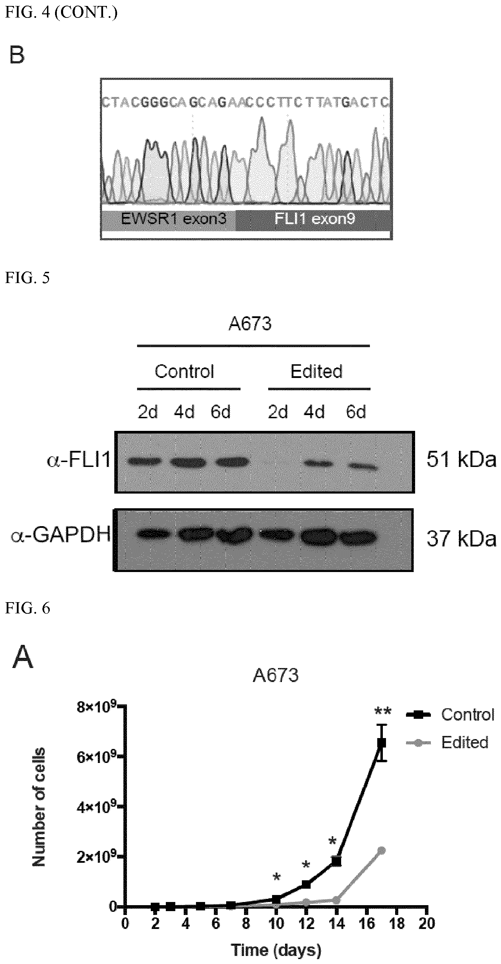

Figure 4. Analysis of the EWSR1-FLI1 RNA. a. Agarose gel electrophoresis of the EWSR1-FLI1 RT-PCR products obtained from edited and control A673 ES cells. RT-PCR analysis was done using RNA from cell cultures on days 2, 4 and 6 pt. Arrows depict the sizes of wild type (961bp) and deleted (150bp) RT-PCR products. GAPDH is used as an internal control of the RT-PCR reaction b. Representative deleted cDNA sequence obtained by Sanger and chromatogram.
Figure 5. Analysis of the EWSR1-FLI1 protein. EWSR1-FLI1 protein expression in A673 ES cells by western blot analysis. Western blot analysis was done using protein from cell cultures on days 3, 6 and 10 pt. GAPDH is used as an internal control of the assay.

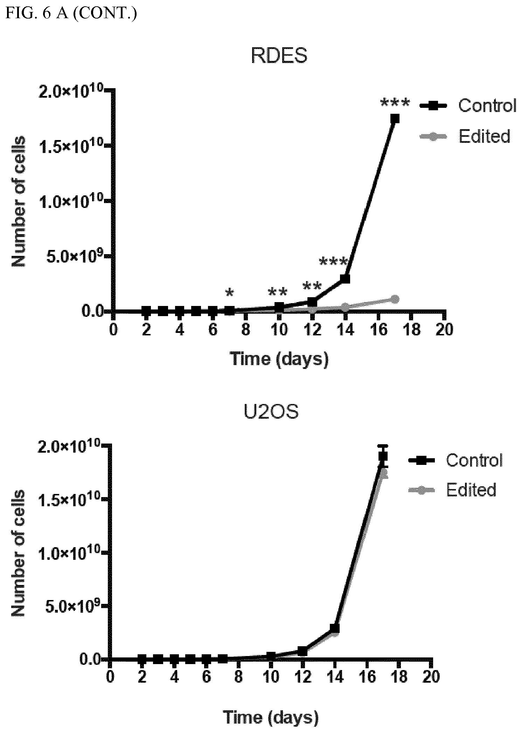

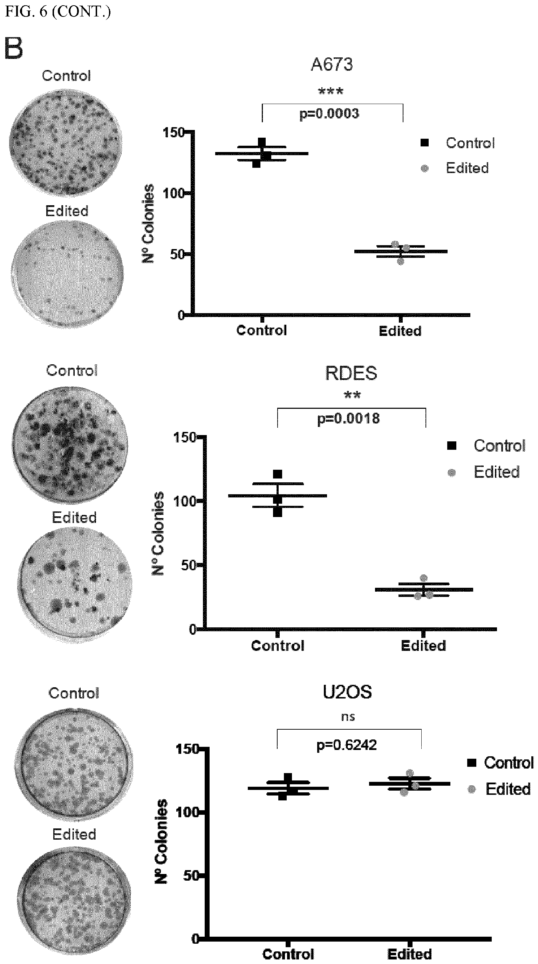

Figure 6. Proliferation and tumorigenicity *in vitro* assays a. Growth rate assay curves of A673 and RD-ES ES and U2OS osteosarcoma (EWSRI-FLI⁻) experimental and control cells. b. Colony formation assay. Representative images of wells after 2% crystal violet staining are shown of the A673, RD-ES and U2OS experimental and control cells. Graphical representation of the number of colonies formed in the A673, RD-ES and U2OS colony formation assays. p values are represented (**p<0.005; ***p<0.0005;).

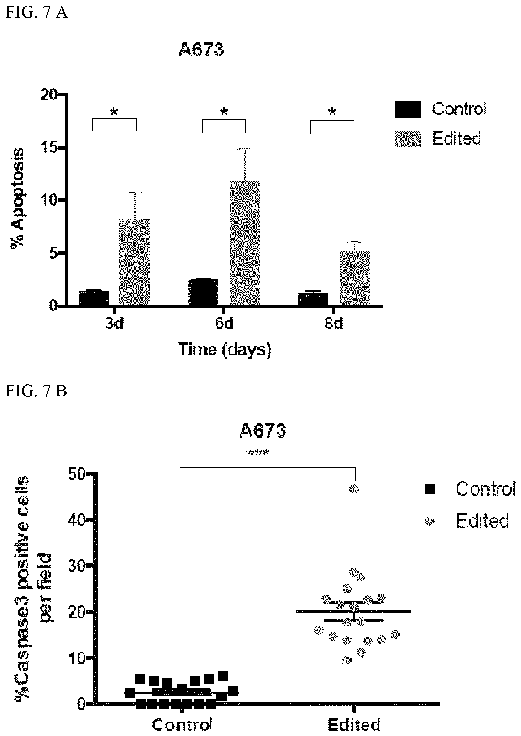

Figure 7. Apoptosis *in vitro* assays. a. The DNA profile was analysed using propidium iodide staining and flow cytometry. The percentage of cellular apoptosis is calculated using the percentage of the Sub-G1 peak. Black plot represent A673 control cells and grey plot represent experimental *EWSR1-FLI1* deleted A673 cells. b. The number of apoptotic cells was analysed using Caspase 3 immunostaining. The percentage of cellular apoptosis is calculated using the percentage of Caspase3 positive cells per field analysed. Black dots represent A673 control cells and grey dots represent experimental *EWSR1-FLI1* deleted A673 cells.

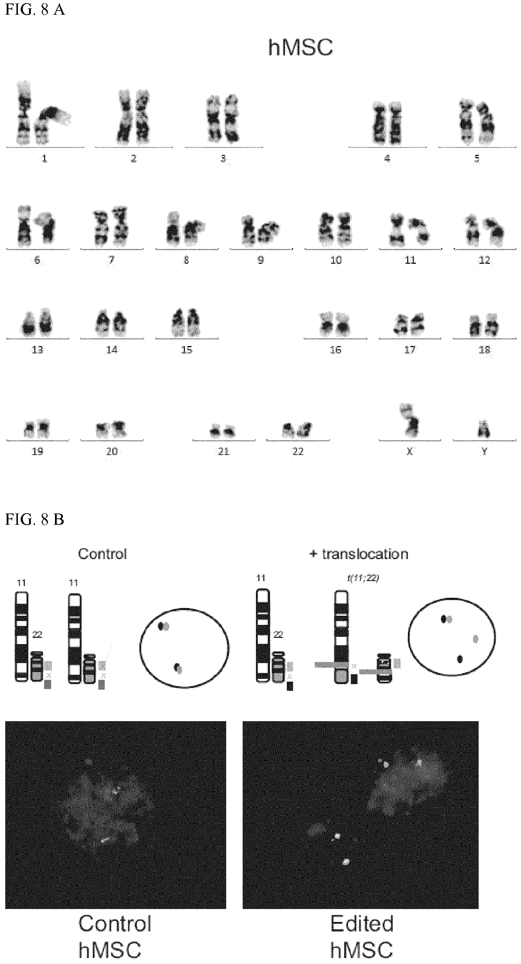

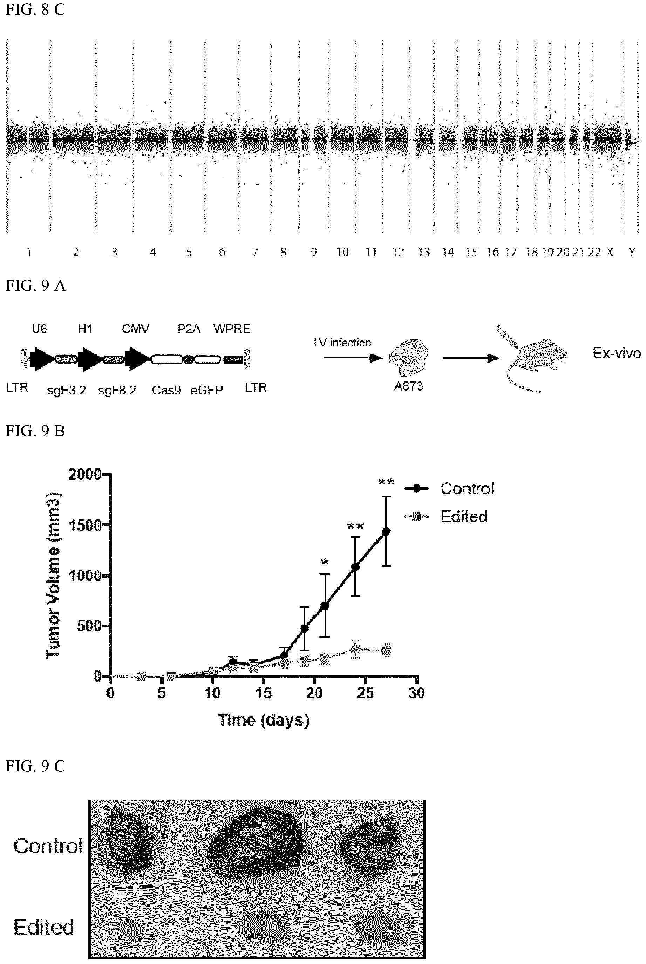

Figure 8. Genome editing specificity analysis. a. Representative G-banded methaphase with normal karyotype of WT human mesenchymal stem cells (hMSC) transduced with sgE3.2 and sgF8.2. b. FISH analysis of EWSR1 gene status. The schematic representation shows the structure and principle of the EWSR1 break-apart fluorescent in situ hybridization (FISH) probe (Kreatech KBI-10750). A break is defined when a fusion signals splits into separate signals. Co-localized fusion signals identify the normal chromosome(s) 22. Representative FISH images of control and experimental hMSCs. c. Profile plot result of a high-density array comparative genomic hybridization (aCGH) analysis covering the whole genome showing no copy number variations (CNVs) in hMSCs transduced with sgE3.2 and sgF8.2.

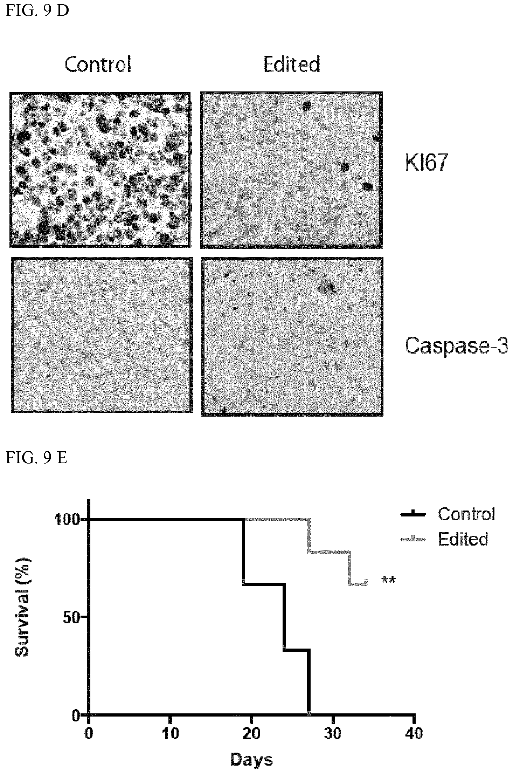

Figure 9: Ex-vivo lentiviral *EWSR1-FLI1* gene edition. a. Diagram showing the lentiviral vector and the approach for the ex-vivo treatment. A673 ES cells are transduced with of the pLVX-U6E3.2-H1F8.2-Cas9-2A-eGFP or control vectors and transplanted into immunocompromided mice. The xenografted mice are then observed and measured and sacrificed 30 days post cell injection. b. Tumour growth (mm3) over the 28 days following subcutaneous cell injection. The plot shows medians and ranges; p values are represented (*p<0.05, **p<0.005). c. Mice were sacrificed after 30 days and their tumours collected. Pictures show representative tumours of control and experimental mice. D. Representative images of Ki-67 proliferation marker and Caspase3 apoptosis marker immunostaining assays on A673 ES experimental and control cells. e. Survival curve comparing mice injected with experimental or control A673 cells.

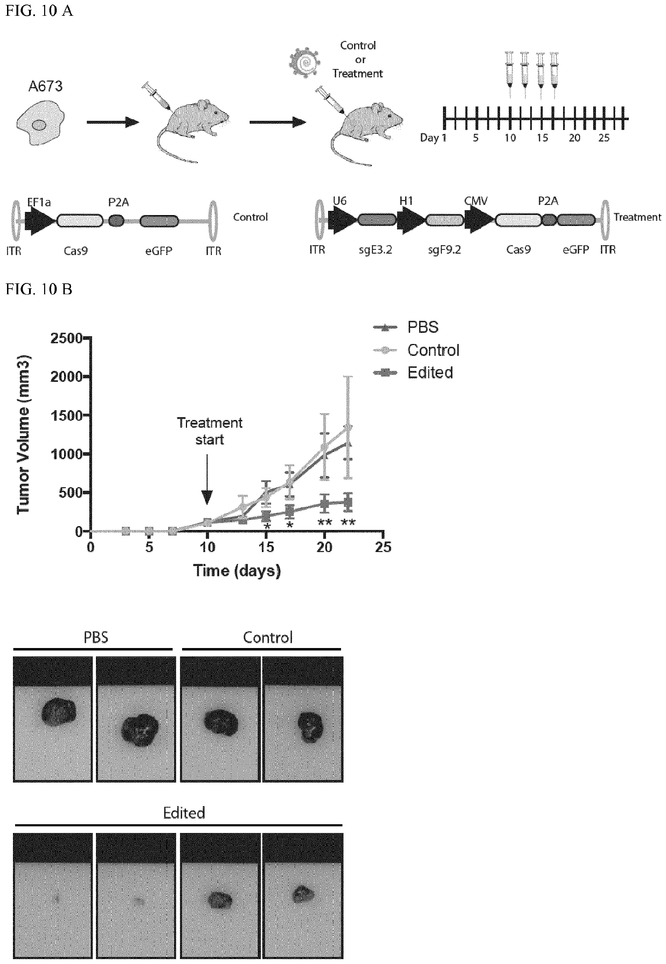

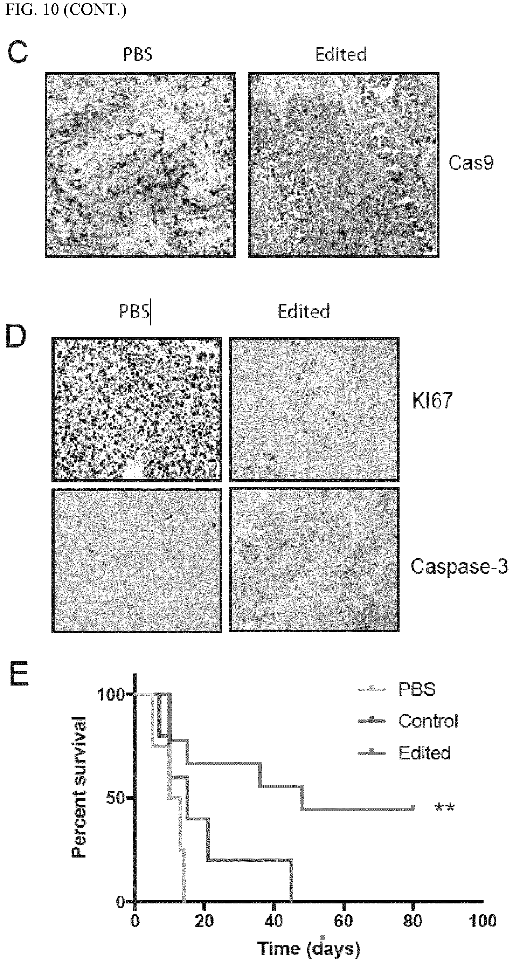

Figure 10: In-vivo adenoviral *EWSR1-FLI1* gene edition. a. Diagram showing the control Cas9 and sgE3.2, sgF8.2 and Cas9 adenoviral vectors and schedule used for the in-vivo gene edition assays. A673 ES cells are injected in the flanks of immunocompromised mice, when reached a defined size the adenoviral, control vector and PBS are injected 4 times (every3 days) in the xenografted tumours. The xenografted mice are then observed and measured and sacrificed 30 days post cell injection. b. Tumour growth (mm3) over the 23 days. The plot shows medians and ranges; p values are represented (*p<0.05, **p<0.005). Mice were sacrificed after 25 days and their tumours collected. Pictures show representative tumours of control and experimental mice. c. Representative images of Cas9 immunostaining assays on A673 Ewing sarcoma experimental and control cells. d. Representative images of Ki-67 proliferation and Caspase3 apoptosis markers immunostaining assays on A673 Ewing sarcoma experimental and control cells e. Survival curve comparing mice treated with sgRNAs-Cas9 experimental adenoviral vector, Cas9 control adenoviral vector or PBS.

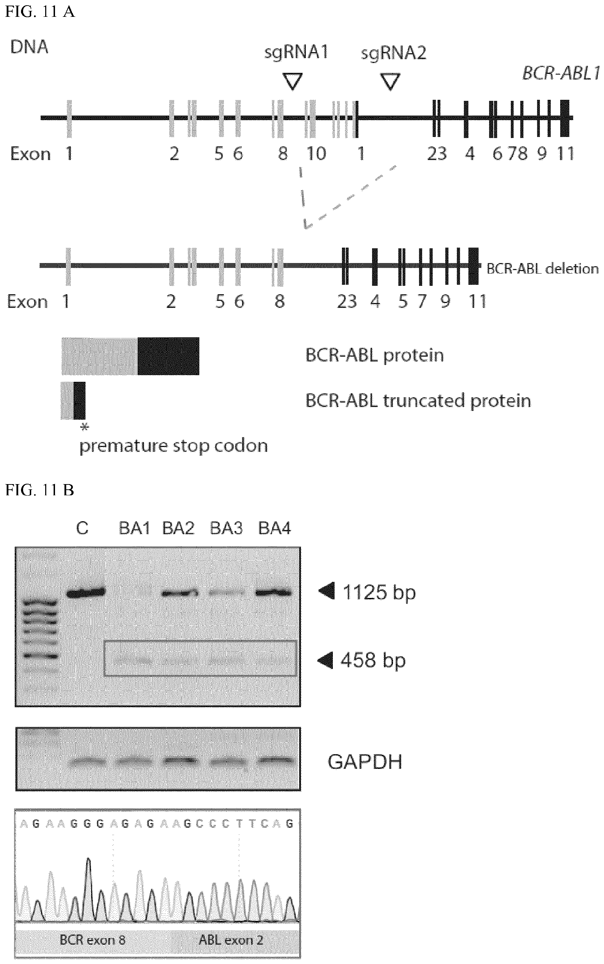

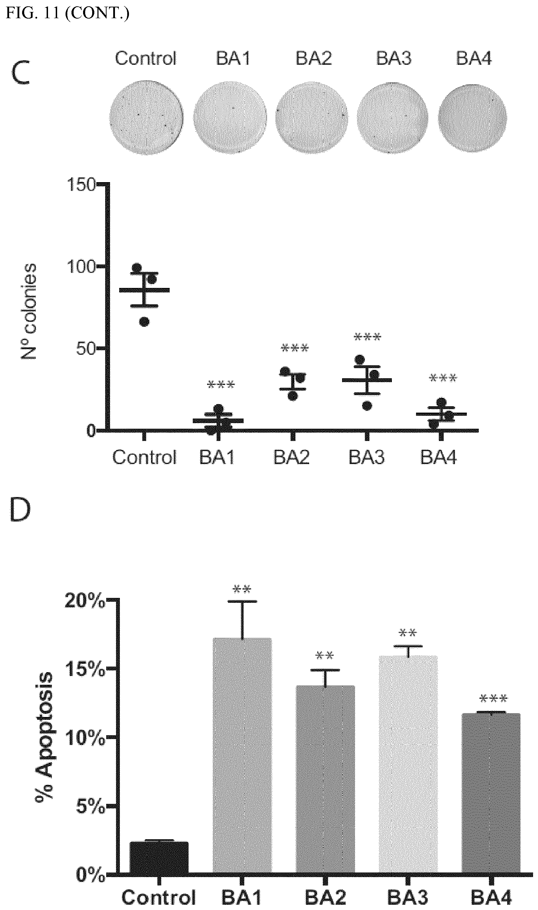

Figure 11: a. Schematic representation of the *BCR-ABL1* fusion gene. Indicated are the sgRNAs targeting introns 8 of *BCR* and 1 of *ABL1* genes used to edit the fusion gene. b. Schematic representation of the BCR-ABL1 chimeric and truncated protein generated by Cas9 edition. b. Amino acid sequence of the BCR-ABL protein (p210). Residues corresponding to BCR or to ABL are shown in black or grey, respectively. ABL DNA binding domain is underlined. Amino acid sequence of the edited BCR-ABL truncated protein. Deleted residues are shown crossed out, the new residues generated by the change of reading frame after the mutation are shown in italics. The premature STOP codon is shown with an asterisk. Analysis of *BCR-ABL1* RNA, and in vitro viability. Four pairs (BA1, BA2, BA3 and BA4) of sgRNAs targeting both BCR and ABL1 introns were tested. b. Agarose gel electrophoresis showing the *BCR-ABL1* RT-PCR products obtained from experimental and control K562 Chronic Myeloid Leukaemia cell line. RT-PCR analysis was done using RNA from cell cultures on day 2 post-nucleofection. Arrows depict the sizes of wild type (1125bp) and deleted (458bp) RT-PCR products. GAPDH is used as an internal control of the RT-PCR reaction. A representative deleted cDNA sequence obtained by Sanger and chromatogram is shown at the bottom. c. Colony formation assay. Representative images of wells after 2 % crystal violet staining are shown of the K562 experimental and control cells. Graphical representation of the number of colonies formed in the K562 colony formation assays. p values are represented (***p<0.0005;). d. Apoptosis *in vitro* assays. The DNA profile was analysed using propidium iodide staining and flow cytometry. The percentage of cellular apoptosis is calculated using the percentage of the Sub-G1 peak. Black plot represent K563 control cells and grey plots represent experimental BA1, BA2, BA3 and BA4 edited K562 cells.

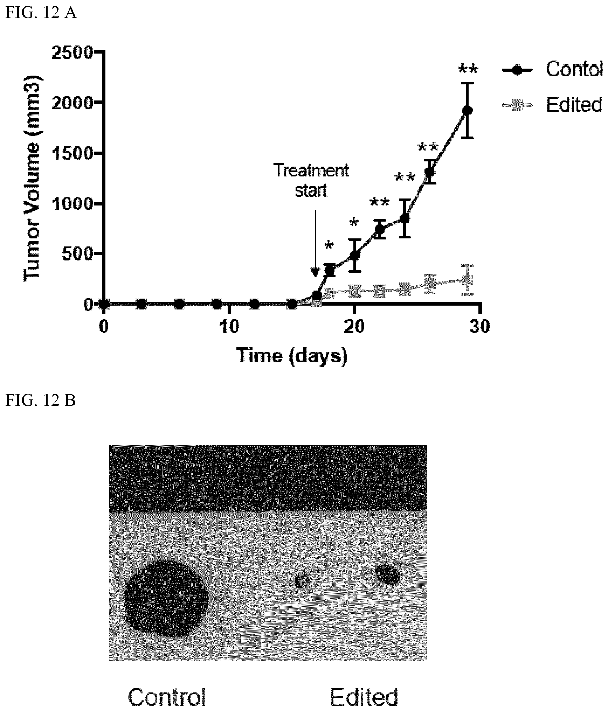

Figure 12. In-vivo adenoviral *BCR-ABL1* gene edition. a. Tumour growth (mm3) over the 30 days following subcutaneous cell injection and 4 in-vivo adenoviral treatments (every3 days). The plot shows medians and ranges; p values are represented (**p<0.05). b. Mice were sacrificed after 30 days and their tumours collected. Pictures show representative tumours of control and experimental mice. c. Survival curve comparing mice treated with sgRNAs-Cas9 experimental and control adenoviral vectors.

### EXAMPLES

### Precise deletion of fusion genes via CRISPR-Cas9.

To elucidate whether the fusion gene deletion (or rearrangement) strategy is a good gene therapy approach to treat cancer, the inventors have chosen as test models two well characterized fusion genes representative of the two major classes of clinically relevant transcription fusions: *EWSR1-FLI1* Ewing's sarcoma (ES) transcription factor and *BCR-ABL* chronic myeloid leukaemia (CML) tyrosine kinase fusion genes.

Ewing's sarcoma (ES), the second most common cancer involving bone in children, is characterized by a chromosomal translocation that fuses the strong transactivation domain of the RNA binding protein *EWSR1,* with the DNA binding domain of an ETS protein, most commonly *FLI1.* EWSR1-FLI1 acts as a transcriptional factor, and numerous studies have demonstrated a strict dependency on *EWSR1-FLI1* expression of ES cells. Two main *EWSR1-FLI1* subtypes have been described, fusing the *EWSR1* exon 7 to *FLI1* exon 6 (so-called type 1) or to *FLI1* exon 5 (so-called type 2 (Figure 1a). Two ES cell lines (A673 and RD-ES) harbouring two different isoforms, type 1 and type2, of the *EWSRI-FLI1* fusion gene have been chosen as model systems.

ES and CML have been selected as test models, but it is important to keep in mind that the overall approach is potentially applicable to all neoplasias addicted to the expression of fusion genes or the enforced expression of oncogenes produce by chromosomal rearrangements whose removal or modification via genome editing would cause death of tumour cells.

### Selection of sgRNAs that target EWSRI-FLI1 fusion gene

To selectively target both isoforms of *EWSRI-FLI1* with the same CRISPR tool, a pair of sgRNAs targeting introns 3 of *LWSR1* and intron 8 of *FLI1* was designed (Figure 1a and Table 2). The targeted introns were selected first, to generate a large deletion including key functional domains of the fusion gene, secondly to induce a frameshift of the remaining 3' region of the FLI1 gene, and thirdly to include all the hotspot introns harbouring breakpoints in human patients. The design of the sgRNAs in intronic regions guaranteed no modification of the wild type EWSR1 and FLI1 proteins in non-tumour cells because any indel generated by the NHEJ repair of the on-target introns in wild type (WT) genes will be removed in the mRNA. This is because the splicing machinery will remove any indel generated in the on-target sites of both sgRNAs in the introns of wild type genes after nonhomologous end-joining (NHEJ) repair of the double strand breaks (DSBs) generated by the Cas9 nuclease (Figure 1c). Consequently, deletions will only take place in those cells harbouring the fusion gene with both on-target intronic regions in the same chromosome (Figure 1a).

**Table 2. sgRNA sequences used for CRISPR-based gene editing. Oligonucleotide sequences used for PCR, RT-PCR and NGS analysis.**

| **sgRNAs** | | | | | |
|---|---|---|---|---|---|
| sgE3.1 | SEQ ID NO: 33 | TAAGAGGACATACAGCGTTC TGG | sgF6.1 | SEQ ID NO: 34 | ATTTGGACCTGTGGCGATAT GGG |
| sgE3.2 | SEQ ID NO: 2 | TGGTTGCACAGTAAGTGGCG GGG | sgF6.2 | SEQ ID NO: 51 | AAGACGTCTTGCTCCCCTCG GGG |
| sgE6.1 | SEQ ID NO: 35 | CGGGCGGATCATATAAGGTC AGG | sgF8.1 | SEQ ID NO: 36 | CAAGTCGATCCCAATGTCGA AGG |
| sgE6.2 | SEQ ID NO: 37 | TAGATCGCGTACTCCATCCT GGG | sgF8.2 | SEQ ID NO: 3 | AGTGGGCCACACTGCGACAA GGG |
| sgB1 | SEQ ID NO: 4 | TATCCGAGGCACGTTAAGGG | sgA1 | SEQ ID NO: 5 | CACGAGGTTGACGCACCAGA |
| sgB2 | SEQ ID NO: 38 | GACATGACCATGGGTAAGCG | sgA2 | SEQ ID NO: 39 | TCCCTAATAGTGATGGCGCT |

| **PCR/RT-PCR EF deletion detection primers** | | | | | |
|---|---|---|---|---|---|
| Ex3 EWSR1 fw | SEQ ID NO: 40 | GCCCAGCCCACTCAAGGATA | Ex9 FLI1 rv | SEQ ID NO: 41 | TTGGGGTTGGGGTAGATTCC |
| qEWSF1 fw | SEQ ID NO: 42 | GCAGGGCTACAGTGCTTAC | qEWSFLI rv | SEQ ID NO: 43 | GCAGCTCCAGGAGGAATTG |

| **On target detection primers** | | | | | |
|---|---|---|---|---|---|
| EWSR1 OT fw | SEQ ID NO: 44 | AGGTGCCCTGTTCCATGCT | EWSR1 OT rv | SEQ ID NO: 45 | AGGTGCCCTGTTCCATGCT |
| FLI1 OT fw | SEQ ID NO: 46 | GTGAGTTTACCTTGGCCTGC | Int8 Fli1 rv | SEQ ID NO: 47 | GTGAGTTTACCTTGGCCTGC |

| **qPCR primers** | | | | | |
|---|---|---|---|---|---|
| qEWSF1 fw | SEQ ID NO: 48 | CCCAGCCAGATCCGTATCAG | qEWSFLI rv | SEQ ID NO: 49 | GCAGCTCCAGGAGGAATTG |
| qEWSF2 fw | SEQ ID NO: 50 | GCAGGGCTACAGTGCTTAC | | | |
| GAPDH fw | SEQ ID NO: 52 | ACCACAGTCCATGCCATCA | GAPDH rv | SEQ ID NO: 53 | TCCACCACCCTGTTGCTGTA |

| **RT-PCR BA deletion detection primers** | | | | | |
|---|---|---|---|---|---|
| qBCR-ABL | SEQ ID | GATGCCAAGGATCCAACGAC | qBCR-ABL rv | SEQ ID | GGCTTCACACCATTCCCCAT |
| fw | NO: 54 | | | NO: 55 | |

| **PCR/RT-PCR controls primers** | | | | | |
|---|---|---|---|---|---|
| Albumin fw | SEQ ID NO: 56 | GCTGTCATCTCTTGTGGGCTGT | Albumin rv | SEQ ID NO: 57 | ACTCATGGGAGCTGCTGGTTC |
| GAPDH fw | SEQ ID NO: 58 | ACCACAGTCCATGCCATCA | GAPDH rv | SEQ ID NO: 59 | TCCACCACCCTGTTGCTGTA |

| **Deep sequencing primers** | | | | | |
|---|---|---|---|---|---|
| EWSR1 ON NGS fw | SEQ ID NO:60 | CTTTCCCTACACGACGCTCTTCCGAT CTAGGTGCCCTGTTCCATGCT | EWSR1 ON NGS rv | SEQ ID NO: 61 | GACTGGAGTTCAGACGTGTGCTCTTCCGATCTTGGCC TAGGCTTTTCAACAGA |
| EWSR1 offT1 NGS fw | SEQ ID NO: 62 | CTTTCCCTACACGACGCTCTTCCGAT CTATGGTATTCTCACGCTGCCA | EWSR1 offT1 NGS rv | SEQ ID NO: 63 | GACTGGAGTTCAGACGTGTGCTCTTCCGATCTTGGA GATGAATGGGAAGCGAA |
| EWSR1 offT2 NGS fw | SEQ ID NO: 64 | CTTTCCCTACACGACGCTCTTCCGAT CTTCCCACTTGTTTATTCCTCTGTG | EWSR1 offT3 NGS rv | SEQ ID NO: 65 | GACTGGAGTTCAGACGTGTGCTCTTCCGATCTATTCC AGAGAAGGACATTGCCA |
| EWSR1 offT3 NGS fw | SEQ ID NO: 66 | CTTTCCCTACACGACGCTCTTCCGAT CTTGGGAGTTCTCTAAGGCTGC | EWSR1 offT3 NGS rv | SEQ ID NO:67 | GACTGGAGTTCAGACGTGTGCTCTTCCGATCTGTGA CTTTTCCCACCGCCTC |
| EWSR1 offT4 NGS fw | SEQ ID NO: 68 | CTTTCCCTACACGACGCTCTTCCGAT CTCTCCTTTTCTCCTCCTGCCAGC | EWSR1 offT4 NGS rv | SEQ ID NO: 69 | GACTGGAGTTCAGACGTGTGCTCTTCCGATCTGACTT GGATCTTCAACCGCC |
| EWSR1 offT5 NGS fw | SEQ ID NO: 70 | CTTTCCCTACACGACGCTCTTCCGAT CTAGCTTGCTATTCTTTGAGATGAAC | EWSR1 offT5 NGS rv | SEQ ID NO: 71 | GACTGGAGTTCAGACGTGTGCTCTTCCGATCTTGAAT AAAGGCCCCGATGACC |
| EWSR1 offT6 NGS fw | SEQ ID NO: 72 | CTTTCCCTACACGACGCTCTTCCGAT CTCCTGGGTTGTAACTGTGGGT | EWSR1 offT6 NGS rv | SEQ ID NO: 73 | GACTGGAGTTCAGACGTGTGCTCTTCCGATCTTTCTG GGAGTCGTAGGCTTAGT |
| EWSR1 offT7 NGS fw | SEQ ID NO: 74 | CTTTCCCTACACGACGCTCTTCCGAT CTCTCGGGCCTGTTCCTTCATA | EWSR1 offT7 NGS rv | SEQ ID NO: 75 | GACTGGAGTTCAGACGTGTGCTCTTCCGATCTCCACC TCCAGAAGCCCTTAG |
| EWSR1 offT8 NGS fw | SEQ ID NO: 76 | CTTTCCCTACACGACGCTCTTCCGAT CTGCAAGAATTTCAAGGCCCCAG | EWSR1 offT8 NGS rv | SEQ ID NO: 77 | GACTGGAGTTCAGACGTGTGCTCTTCCGATCTAGGG ATGACTTGACTGCTGA |
| EWSR1 offT9 NGS fw | SEQ ID NO: 78 | CTTTCCCTACACGACGCTCTTCCGAT CTGTCACTCACCTGGCTGCTTC | EWSR1 offT9 NGS rv | SEQ ID NO: 79 | GACTGGAGTTCAGACGTGTGCTCTTCCGATCTAGCA TTCCTCATTTGATTCCAGA |
| FLI1 ON NGS fw | SEQ ID NO: 80 | CTTTCCCTACACGACGCTCTTCCGAT CTGTTGTCTCCCGCATGCCAG | FLI1 ON NGS rv | SEQ ID NO: 81 | GACTGGAGTTCAGACGTGTGCTCTTCCGATCTGGAA TGGGTAGGCAGAGTC |
| FLI1 offT1 NGS fw | SEQ ID NO: 82 | CTTTCCCTACACGACGCTCTTCCGAT CTAGGGAGGGTCTAATCTAGGAGC | FLI1 offT1 NGS rv | SEQ ID NO: 83 | GACTGGAGTTCAGACGTGTGCTCTTCCGATCTCCCTC TTCCCCACCATTTTGT |
| FLI1 offT2 NGS fw | SEQ ID NO: 84 | CTTTCCCTACACGACGCTCTTCCGAT CTGCATACAGGGCTTCTTTCGTG | FLI1 offT2 NGS rv | SEQ ID NO: 85 | GACTGGAGTTCAGACGTGTGCTCTTCCGATCTCGTTC TTCCTGTGCCATCCT |
| FLI1 offT3 NGS fw | SEQ ID NO: 86 | CTTTCCCTACACGACGCTCTTCCGAT CTTGTGTGGAGGAGGGAGTCAA | FLI1 offT3 NGS rv | SEQ ID NO: 87 | GACTGGAGTTCAGACGTGTGCTCTTCCGATCTGGCCT TCAGAACTCATCAAGG |
| FLI1 offT4 NGS fw | SEQ ID NO: 88 | CTTTCCCTACACGACGCTCTTCCGAT CTATCCTCACAGAGCATTGCAG | FLI1 offT4 NGS rv | SEQ ID NO: 89 | GACTGGAGTTCAGACGTGTGCTCTTCCGATCTGTACT GATTCTGGGGCTTGCT |
| FLI1 offT5 NGS fw: | SEQ ID NO: 90 | CTTTCCCTACACGACGCTCTTCCGAT CTCGTTGGCTGTGTGTCTGTTTC | FLI1 offT5 NGS rv | SEQ ID NO: 91 | GACTGGAGTTCAGACGTGTGCTCTTCCGATCTAGGA GTGGGGAGTCTTTCGT |
| FLI1 offT6 NGS fw: | SEQ ID NO: 92 | CTTTCCCTACACGACGCTCTTCCGAT CTAGGAGACCGATGGACAGACG | FLI1 offT6 NGS rv | SEQ ID NO: 93 | GACTGGAGTTCAGACGTGTGCTCTTCCGATCTCCTCC CTCCTTTCCCCTGAC |
| FLI1 offT7 NGS fw: | SEQ ID NO: 94 | CTTTCCCTACACGACGCTCTTCCGAT CTTCCATAAGTTGACTCTGGCAGG | FLI1 offT7 NGS rv | SEQ ID NO: 95 | GACTGGAGTTCAGACGTGTGCTCTTCCGATCTAGAG TGCCTTGGTCAAATGG |
| FLI1 offT8 NGS fw: | SEQ ID NO: 96 | CTTTCCCTACACGACGCTCTTCCGAT CTAGTGTTGGGATTACAGGCGTG | FLI1 offT8 NGS rv | SEQ ID NO: 97 | GACTGGAGTTCAGACGTGTGCTCTTCCGATCTGCCTG GGAATTTCACTGTGCC |
| FLI1 offT9 NGS fw: | SEQ ID NO: 98 | CTTTCCCTACACGACGCTCTTCCGAT CTAGTTCCCCTCTCCTCCCTG | FLI1 offT9 NGS rv | SEQ ID NO: 99 | GACTGGAGTTCAGACGTGTGCTCTTCCGATCTCACTT CCCATGGACAGCTTG |
| FLI1 offT10 NGS fw: | SEQ ID NO: 100 | CTTTCCCTACACGACGCTCTTCCGAT CTGAGGGGTAAAGGATTGGAGCC | FLI1 offT10 NGS rv | SEQ ID NO: 101 | GACTGGAGTTCAGACGTGTGCTCTTCCGATCTCGGA GAGATTGAAGGGAGCG |

| **crRNAs** | | | | | |
|---|---|---|---|---|---|
| EWSR1-FLI1 type I | SEQ ID NO: 102 | GTCATAAGAAGGGTTCTGCTGCCCGT AG | | | |
| EWSR1-FLI1 type II | SEQ ID NO: 103 | GGCCAGCAGTGAACTCTGCTGCCCGT AG | | | |
| BCR-ABL | SEQ ID NO: 104 | CCGCTGAAGGGCTTTTGAACTCTGCT TA | | | |

A couple of sgRNAs were designed for each intronic region using the crispr.mit.edu/ and benchling.com/crispr webtools following the standard sgRNA design principles: making sure to pick targeting sequences that are upstream of a PAM sequence, unique to the target compared to the rest of the genome, and selecting those with as few predicted off-target events as possible. The sgRNAs, *sgEWSR13.2* (hereafter sgE3.2 (SEQ ID NO: 2)) and sg*FLI1*8.2 (sgF8.2 (SEQ ID NO: 3)) were cloned in the pLVX-U6E3.2-HIF8.2-Cas9-2A-eGFP (hereafter pLV-U6EH1F-C9G) that drives similar sgRNA expression levels from two different RNA polymerase III promoters (U6 and HI) and a simultaneously regulated expression of Cas9 and GFP proteins by a 2A self-cleaving peptide (Figure Id). Cleavage of introns 3 and 8 of *EWSR1* and *FLI1,* respectively, should result in a deletion of 27.67kb, removing a critical portion of the *LWSR1* transactivation domain, and together with a frameshift alteration of the entire *FLI1* DNA binding domain (Figure 1b and 2). SEQ ID NO: 6 is the amino acid sequence of the EWSR1-FLI1 chimeric protein and SEQ ID NO: 7 is the amino acid sequence of the edited EWSR1-FLI1 truncated protein, both represented in figure 2. A673 cells were transduced with the pLV-U6EH1F-C9G vector and total genomic DNA was isolated at 2, 4 and 6 days post-transduction (pt) for subsequent analysis. 2 days pt DNA deep sequencing analysis showed indel frequencies of 61,8 in *EWSR1* and 66,2% in *FLI1* on-target sites (table 3).

Table 3. NGS analysis of the on-target EWSR1 and FLI1 sites. a,c. Summary of the *LWSR1* and *FLI1* loci analysis (sgRNA sequence, chromosome position, total reads and efficiency). b,d. Indels at *EWSR1* and *FLI1* loci in induced A673 edited cells. Wild-type (WT) sequences are listed at the top of each figure. sgRNA sequence is underlined Identified mutations are shown in bold font. -, deletion.

**a. (SEQ ID NO: 2)**

| | | | | Control | | | Edited | | |
|---|---|---|---|---|---|---|---|---|---|
| **On Target:** | **sgRNA sequence (5'-3')** | **Chromosome** | **Position** | **Total reads** | **Modified Reads** | **Editing efficiency (%)** | **Total reads** | **Modified Reads** | **Editing efficiency (%)** |
| **sgEWS32** | **TGGTTGCACAGTAAGTGGCG** | 22 | 29272832 | 49650 | 0 | 0,0 | 22981 | 14207 | 61,8 |

**b. (SEQ ID NOs: 8 to 19)**

| Sequence | Reads |
|---|---|
| GCAGTGCATAGATATTAAGTAACTTGCCAGTGGTTGCACAGTAAGTGGCGGGGTTAGCTCTAAAAACTGGCGACCTAGCCAA | x7858 |
| GCAGTGCATAGATATTAAGTAACTTGCCAGTGGTTGCACAGTAAGT-GCGGGGTTAGCTCTAAAAACTGGCGACCTAGCCAA | x1459 |
| GCAGTGCATAGATATTAAGTAACTTGCCAGTGGTTGCACAGTAAGT**GTG**GCGGGGTTAGCTCTAAAAACTGGCGACCTAGCCAA | x835 |
| GCAGTGGCATAGATATTAAGTAACTTGCCAGTGGTTGCACAGT**GG**GCGGGGTTAGCTCTAAAAACTGGCGACCTAGCCAA | x763 |
| GCAGTGCATAGATATTAAGTAACTTGCCAGTGGTTGCACAGTAA---GCGGGGTTAGCTCTAAAAACTGGCGACCTAGCCAA | x688 |
| GCAGTGCATAGATATTAAGTAACTTGCCAGTGGTT------------GCGGGGTTAGCTCTAAAAACTGGCGACCTAGCCAA | x526 |
| GCAGTGCATAGATATTAAGTAACTTGCCAGTGGTTGCACAGTAA---GGCGGGGTTAGCTCTAAAAACTGGCGACCTAGCCAA | x446 |
| GCAGTGCATAGATATTAAGTAACTTGCC---------------AGTGGCGGGGTTAGCTCTAAAAACTGGCGACCTAGCCAA | x343 |
| GCAGTGCATAGATATTAAGTAACTTGCCAGTGGTTGCACA-------GCGGGGTTAGCTCTAAAAACTGGCGACCTAGCCAA | x286 |
| GCAGTGCATAGATATTAAGTAACTTGCCAGTGGTTGCACA------------GTTAGCTCTAAAAACTGGCGACCTAGCCAA | x254 |
| GCAGTGCATAGATATTAAGTAACTTGCCAGT--------------------GGTTAGCTCTAAAAACTGGCGACCTAGCCAA | x218 |
| GCAGTGCATAGATATTAAGTAACTTGCCAGTGGTTGCACAGTA----GCGGGGTTAGCTCTAAAAACTGGCGACCTAGCCAA | x213 |

**c. (SEQ ID NO: 3)**

| | | | | **Control** | | | **Edited** | | |
|---|---|---|---|---|---|---|---|---|---|
| **On Target:** | **sgRNA sequence (5'-3')** | **Chromosome** | **Position** | **Total reads** | **Modified Reads** | **Editing efficiency (%)** | **Total reads** | **Modified Reads** | **Editing efficiency (%)** |
| sgFLI8.2 | AGTGGGCCACACTGCGACAA | 11 | 128809772 | 1461 | 0 | 0,0 | 12315 | 8151 | 66,2 |

**d. (SEQ ID NOs: 20 to 31)**

| Sequence | Reads |
|---|---|
| TGGGTAGGCAGAGTCCCTGGGATGGGAAGGTGAGTGGGCCACACTGCGACAAGGCCTGCTAGCTCCCAATCTCGATGGACTT | x3792 |
| TGGGTAGGCAGAGTCCCTGGGATGGGAAGGTGAGTGGGCCACACTGCGA**A**CAAGGGCCTGCTAGCTCCCAATCTCGATGGACTT | x1111 |
| TGGGTAGGCAGAGTCCCTGGGATGGGAAGGTGAGTGGGCCACACTGC-------------TAGCTCCCAATCTCGATGGACTT | x622 |
| TGGGTAGGCAGAGTCCCTGGGATGGGAAGGTGAGTGGGCC-----------------TGCTAGCTCCCAATCTCGATGGACTT | x515 |
| TGGGTAGGCAGAGTCCCTGGGATGGGAAGGTGAGTGGGCCACACTGCGA-AAGGGCCTGCTAGCTCCCAATCTCGATGGACTT | x502 |
| TGGGTAGGCAGAGTCCCTGGGATGGGAAGGTGAGTGGGCCACACTGCGA---GGGCCTGCTAGCTCCCAATCTCGATGGACTT | x411 |
| TGGGTAGGCAGAGTCCCTGGGATGGGAAGGTGAGTGGCCACACTGC---AAGGGCCTGCTAGCTCCCAATCTCGATGGACTT | x326 |
| TGGGTAGGCAGAGTCCCTGGGATGGGAAGGTGAGTGGGCCACACTGCGA--AGGGCCTGCTAGCTCCCAATCTCGATGGACTT | X197 |
| TGGGTAGGCAGAGTCCCTGGGATGGGAAGGTGAGTGGGCCACACTGCGA---------GCTAGCTCCCAATCTCGATGGACTT | x191 |
| TGGGTAGGCAGAGTCCCTGGGATGGGAAGGTGAGTGGGCCACACTG-----------------CTCCCAATCTCGATGGACTT | x173 |
| TGGGTAGGCAGAGTCCCTGGGATGGGAAGGTGAGTGGGCCACACTGCG-CAAGGGCCTGCTAGCTCCCAATCTCGATGGACTT | x155 |
| TGGGTAGGCAGAGTCCCTGGGATGGGAAGGTGAGTGGGCCACA--------AGGGCCTGCTAGCTCCCAATCTCGATGGACTT | x147 |

### Targeting the EWSR1-FLI1 fusion gene in vitro

Two ES cell lines, A673 and RD-ES, harbouring respectively the type1 or type2 *EWSR1-FLI1* isoforms, were chosen as model systems. We first examined the ability of pLV-U6EH1F-C9G to generate *EWSR1-FLI1* fusion gene deletions in the A673. Osteosarcoma U2OS cell line, which do not contain the fusion gene and an empty pLV-U6#H1#-C9G vector that do not contain sgRNA sequences were used as cell line and vector controls, respectively. PCR analysis of genomic DNA region spanning the intronic target sites extracted at days 2, 4 and 6 pt revealed a unique Δ27.67kb deletion product whose sequence was verified by Sanger sequencing (Figure 3). Similarly, the simultaneous sgE3.2 and sgF8.2 expression resulted in a robust reduction of EWSR1-FLI1 mRNA and protein, as showed by qRT-PCR and Western blot analysis (Figures 4 and 5).

### Targeting EWSRI-FLI1 fusion gene with a KO deletion CRISPR-based approach inhibits cancer cell survival, proliferation and tumorigenicity in vitro

To investigate whether targeted deletion of *EWSRI-FLI1* could induce death of cancer cells, cell survival, proliferation and tumorigenicity *in vitro* assays were conducted. A673, RD-ES and U2OS transduced with pLV-U6EH1F-C9G and control plasmid were subjected to growth rate and colony forming on soft agar assays. The growth rate assay demonstrated that *EWSR1-FLI1* deletion in both A673 and RD-ES significantly suppressed cell proliferation compared with their corresponding control cells and has no effect on U2OS cells (Figure 6a). Furthermore, co-expression of Cas9 and sgRNAs in A673 and RD-ES cells, led to 61.5 % and 73.3 % significant reductions in the number of colonies in colony forming and soft agar assays, respectively, suggesting that deletion of *EWSR1-FLI1* using CRISPR inhibits the survival and tumorigenicity of the ES cancer cells. The expression of Cas9 and sgRNAs in U2OS cells did not alter significantly the number of colonies (Figure 6b). Taken together, our data show that *EWSR1-FLI1* deletion insufficient for tumour suppressor activity, inhibiting cell growth. Notably, we observed that 3, 6 and 8 days pt *EWSR1-FLI1* deletion was followed by significant increased cell death, with a peak of 20 % of increased cell death at day 6 measured by the number of cells presenting fragmented DNA (subG1 peak) compared with A673-pLV-U6#H1#-C9G control cells (Figure 7a). Casp3 activation analysis confirmed these results (Figure 7b). Thus, fusion gene deletion leads to apoptotic death in ES cancer cells.

### EWSR1-FLI1 fusion-gene targeting is highly specific

Karyotype and FISH analysis were performed in human mesenchymal stem cells (hMSC) transduced with pLV-U6EH1F-C9G to evaluate whether the cleavage of *EWSR1* and *FLI1* wild type genes could induce genomic alterations in WT cells. G-banded methaphases showed a normal karyotype (Figure 8a); and additionally FISH analysis with an EWSR1 break-apart probe showed no altered FISH signals (Figure 8b). Similarly, high-density array comparative genomic hybridization (aCGH) analysis covering the whole genome showed no copy number variations (CNVs) (Figure 8c). These data suggest that the therapeutic targeting of the cancer *EWSR1-FLI1* fusion gene is highly specific and would not interfere with WT cells. On the other hand, to potentially identify mutations induced at off-target sites we performed next generation sequencing (NGS) of amplicons of 19 genomic regions with the highest homology to the target site (off-target sites 1-9/10 for *EWSR1* and *FLI1,* respectively) at day 7 post-transduction of A673, RD-ES and hMSC cells. None of all read has mutations at the predicted off-target sites (table 4).

### Targeted EWSR1-FLI1 fusion gene deletion induce partial remission of xenografted tumours

In order to determine the effects of *EWSR1-FLI1* deletion *in vivo,* the flanks of nude mice were subcutaneously implanted with control-pLV-U6#H1#-C9G and pLV-U6EH1F-C9G transduced A673 cells (Figure 9a). Mice injected with control cells showed an exponential growth of tumours during the 28 days of analysis. In contrast, mice injected with pLV-U6EH1F-C9G cells gave rise to dramatically smaller subcutaneous tumours than those produced by A673 control cells (Figure 9b and c). Moreover, ex vivo CRISPR edited cells exhibited a markedly reduced number of viable tumour cells and more extensive necrotic regions in CRISPR edited cells than in controls as shown by H&E (hematoxylin and eosin) staining revealed, a significant 65 % reduction of the KI67 proliferation marker and a significant 25 % increased level of caspase-3 protein expression compared with control tumours (Figure 9d). Importantly, mice xenografted with pLV-U6EH1F-C9G cells showed no associated mortality in the 35 days of the study, whereas all controls needed to be sacrificed after two weeks of cell injection (Figure 9e). These results confirmed the therapeutic efficacy of the *EWSR1-FLI1* ex vivo fusion gene edition.

**Table 4. Indel analysis of the most probable off-target sites with the highest homology to the on-target sites by amplicon based next-generation sequencing (NGS) analysis at day 7 pt of hMSC, A673 and RDES cells. On-target sequences are listed at the top of each pannel. Differences in nucleotides with on-target sequence are shown in bold font. None of all read has mutations at the predicted off-target sites.**

| | | | | | 2H WT | | | 2H EF | | |
|---|---|---|---|---|---|---|---|---|---|---|
| OFF Target 2H: | SEQ ID NO: | sgRNA sequence (5-3') | Chrom osome | Position | Total reads | Modified Reads | Editing efficiency (%) | Total reads | Modified Reads | Editing efficiency (%) |
| sgEWS3.2 | 105 | TGGTTGCACAGTAAGTGGCG | | | | | | | | |
| OFT#1 | 106 | TGATTGCACTGTAAGTGGCC | chr5 | -78568232 | 49650 | 2 | 0,0 | 23200 | 5 | 0,0 |
| OFT#2 | 107 | TAGGTGCTCAGTAAGTGGCT | chr10 | -35792862 | 21646 | 0 | 0,0 | 21646 | 1 | 0,0 |
| OFT#3 | 108 | CGACTTCACAGTAAGTGGCG | chr18 | -74446599 | 39768 | 0 | 0,0 | 39768 | 0 | 0,0 |
| OFT#4 | 109 | AGGTAGCGCAGTTAGTGGCG | chr9 | 14347518 | 47792 | 0 | 0,0 | 31603 | 0 | 0,0 |
| OFT#5 | 110 | AGGTAGAACAGTAAGTGGCA | chrl | 194593114 | 3010 | 0 | 0,0 | 27290 | 3 | 0,0 |
| OFT#6 | 111 | TAGTTGTTCAGTAAGTGGCA | chrl | -11805340 | 47295 | 5 | 0,0 | 47691 | 3 | 0,0 |
| OFT#7 | 112 | GGGTAGCAGAGGAAGTGGCG | chr19 | 32779534 | 2985 | 0 | 0,0 | 40221 | 3 | 0,0 |
| OFT#8 | 113 | TGGATGCTGAGTAAGTGGCC | chrX | 115433334 | 550 | 0 | 0,0 | 37235 | 3 | 0,0 |
| OFT#9 | 114 | TGCTTGCAAGGTAAGTGGCC | chr2 | 162011934 | 2487 | 0 | 0,0 | 36328 | 6 | 0,0 |
| sgFLI8.2 | 115 | AGTGGGCCACACTGCGACAA | | | | | | | | |
| OFT#1 | 116 | GATTGGCCACACTGTGACAA | chr9 | -124911364 | 30160 | 8 | 0,0 | 29771 | 10 | 0,0 |
| OFT#2 | 117 | GGCGGGGCACACAGCGACAA | chrl | 20716449 | 57401 | 2 | 0,0 | 23356 | 0 | 0,0 |
| OFT#3 | 118 | AGTGAGGAACACTGCGGCAA | chr6 | 30452057 | 988 | 0 | 0,0 | 25067 | 0 | 0,0 |
| OFT#4 | 119 | TGTGGGCCAGGCTGCGGCAA | chr14 | 58819512 | 480 | 0 | 0,0 | 31988 | 2 | 0,0 |
| OFT#5 | 120 | AGTGGGCTAGCCTGCGACAG | chr10 | 3167786 | 1157 | 0 | 0,0 | 28473 | 2 | 0,0 |
| OFT#6 | 121 | AGTGGGGCTGTCTGCGACAA | chr11 | 793219 | 27414 | 0 | 0,0 | 13965 | 2 | 0,0 |
| OFT#7 | 122 | TGTGAACCACACTGTGACAA | chrX | 126483786 | 30402 | 0 | 0,0 | 7328 | 0 | 0,0 |
| OFT#8 | 123 | AGTGTGCTCCACTGTGACAA | chr18 | 23078807 | 632 | 0 | 0,0 | 724 | 0 | 0,0 |
| OFT#9 | 124 | CGTGGGCCAGCCTGGGACAA | chrX | -153625648 | 19552 | 2 | 0,0 | 8569 | 0 | 0,0 |
| OFT#10 | 125 | AGAGAGCCACACTGAGACAG | chr5 | 133765919 | 64679 | 0 | 0,0 | 34737 | 13 | 0,0 |

| | | | | | A673 WT | | | A673 EF | | |
|---|---|---|---|---|---|---|---|---|---|---|
| OFF Target A673: | SEQ ID NO : | sgRNA sequence (5'-3') | Chromosome | Position | Total reads | Modified Reads | Editing efficiency (%) | Total reads | Modified Reads | Editing efficiency (%) |
| **sgEWS3.2** | 105 | **TGGTTGCACAGTAAGTGGCG** | | | | | | | | |
| OFT#1 | 106 | TGATTGCACTGTAAGTGGCC | chr5 | -78568232 | 1542 | 0 | 0,0 | 16010 | 1 | 0,0 |
| OFT#2 | 107 | TAGGTGCTCAGTAAGTGGCT | chr10 | -35792862 | 21610 | 0 | 0,0 | 32765 | 0 | 0,0 |
| OFT#3 | 108 | CGACTTCACAGTAAGTGGCG | chr18 | -74446599 | 26865 | 0 | 0,0 | 6309 | 0 | 0,0 |
| OFT#4 | 109 | AGGTAGCGCAGTTAGTGGCG | chr9 | 14347518 | 12091 | 0 | 0,0 | 19888 | 0 | 0,0 |
| OFT#5 | 110 | AGGTAGAACAGTAAGTGGCA | chrl | 194593114 | 4982 | 0 | 0,0 | 28486 | 4 | 0,0 |
| OFT#6 | 111 | TAGTTGTTCAGTAAGTGGCA | chrl | -11805340 | 31235 | 2 | 0,0 | 60048 | 2 | 0,0 |
| OFT#7 | 112 | GGGTAGCAGAGGAAGTGGCG | chr19 | 32779534 | 42436 | 4 | 0,0 | 62331 | 2 | 0,0 |
| OFT#8 | 113 | TGGATGCTGAGTAAGTGGCC | chrX | 115433334 | 36814 | 0 | 0,0 | 60048 | 2 | 0,0 |
| OFT#9 | 114 | TGCTTGCAAGGTAAGTGGCC | chr2 | 162011934 | 34218 | 9 | 0,0 | 30383 | 0 | 0,0 |
| **sgFLI8.2** | 115 | **AGTGGGCCACACTGCGACAA** | | | | | | | | |
| OFT#1 | 116 | GATTGGCCACACTGTGACAA | chr9 | -124911364 | 35337 | 4 | 0,0 | 46550 | 7 | 0,0 |
| OFT#2 | 117 | GGCGGGGCACACAGCGACAA | chrl | 20716449 | 7774 | 0 | 0,0 | 1324 | 0 | 0,0 |
| OFT#3 | 118 | AGTGAGGAACACTGCGGCAA | chr6 | 30452057 | 40653 | 4 | 0,0 | 9737 | 2 | 0,0 |
| OFT#4 | 119 | TGTGGGCCAGGCTGCGGCAA | chr14 | 58819512 | 32163 | 0 | 0,0 | 40165 | 0 | 0,0 |
| OFT#5 | 120 | AGTGGGCTAGCCTGCGACAG | chr10 | 3167786 | 39513 | 0 | 0,0 | 8248 | 9 | 0,1 |
| OFT#6 | 121 | AGTGGGGCTGTCTGCGACAA | chr11 | 793219 | 21076 | 2 | 0,0 | 2091 | 0 | 0,0 |
| OFT#7 | 122 | TGTGAACCACACTGTGACAA | chrX | 126483786 | 14647 | 0 | 0,0 | 22616 | 0 | 0,0 |
| OFT#8 | 123 | AGTGTGCTCCACTGTGACAA | chr18 | 23078807 | 2820 | 0 | 0,0 | 3019 | 0 | 0,0 |
| OFT#9 | 124 | CGTGGGCCAGCCTGGGACAA | chrX | -153625648 | 10867 | 0 | 0,0 | 6433 | 0 | 0,0 |
| OFT#10 | 125 | AGAGAGCCACACTGAGACAG | chr5 | 133765919 | 26725 | 9 | 0,0 | 5907 | 0 | 0,0 |

| | | | | | **RDES WT** | | | RDES EF | | |
|---|---|---|---|---|---|---|---|---|---|---|
| **OFF Target RDES** | SEQ ID NO: | **sgRNA sequence (5'-3')** | **Chro moso me** | **Position** | **Total reads** | **Modifie d Reads** | **Editing efficiency (%)** | **Total reads** | Modified Reads | **Editing efficiency (%)** |
| **sgEWS3.2** | 105 | **TGGTTGCACAGTAAGTGGCG** | | | | | | | | |
| OFT#1 | 106 | TGATTGCACTGTAAGTGGCC | chr5 | -78568232 | 14507 | 0 | 0,0 | 22462 | 3 | 0,0 |
| OFT#2 | 107 | TAGGTGCTCAGTAAGTGGCT | chr10 | -35792862 | 32140 | 0 | 0,0 | 22019 | 5 | 0,0 |
| OFT#3 | 108 | CGACTTCACAGTAAGTGGCG | chr18 | -74446599 | 35951 | 4 | 0,0 | 36436 | 0 | 0,0 |
| OFT#4 | 109 | AGGTAGCGCAGTTAGTGGCG | chr9 | 14347518 | 13125 | 0 | 0,0 | 16382 | 5 | 0,0 |
| OFT#5 | 110 | AGGTAGAACAGTAAGTGGCA | chr1 | 194593114 | 5559 | 0 | 0,0 | 27713 | 2 | 0,0 |
| OFT#6 | 111 | TAGTTGTTCAGTAAGTGGCA | chr1 | -11805340 | 57216 | 0 | 0,0 | 53673 | 3 | 0,0 |
| OFT#7 | 112 | GGGTAGCAGAGGAAGTGGCG | chr19 | 32779534 | 27412 | 0 | 0,0 | 40461 | 0 | 0,0 |
| OFT#8 | 113 | TGGATGCTGAGTAAGTGGCC | chrX | 115433334 | 1017 | 0 | 0,0 | 59512 | 2 | 0,0 |
| OFT#9 | 114 | TGCTTGCAAGGTAAGTGGCC | chr2 | 162011934 | 32978 | 0 | 0,0 | 40946 | 0 | 0,0 |
| **sgFLI8.2** | 115 | **AGTGGGCCACACTGCGACAA** | | | | | | | | |
| OFT#1 | 116 | GATTGGCCACACTGTGACAA | chr9 | -124911364 | 23984 | 6 | 0,0 | 24215 | 0 | 0,0 |
| OFT#2 | 117 | GGCGGGGCACACAGCGACAA | chr1 | 20716449 | 2140 | 0 | 0,0 | 42322 | 4 | 0,0 |
| OFT#3 | 118 | AGTGAGGAACACTGCGGCAA | chr6 | 30452057 | 86311 | 0 | 0,0 | 55558 | 5 | 0,0 |
| OFT#4 | 119 | TGTGGGCCAGGCTGCGGCAA | chr14 | 58819512 | 185 | 0 | 0,0 | 31686 | 10 | 0,0 |
| OFT#5 | 120 | AGTGGGCTAGCCTGCGACAG | chr10 | 3167786 | 22928 | 0 | 0,0 | 27899 | 0 | 0,0 |
| OFT#6 | 121 | AGTGGGGCTGTCTGCGACAA | chr11 | 793219 | 9604 | 0 | 0,0 | 30315 | 2 | 0,0 |
| OFT#7 | 122 | TGTGAACCACACTGTGACAA | chrX | 126483786 | 22152 | 10 | 0,0 | 14962 | 0 | 0,0 |
| OFT#8 | 123 | AGTGTGCTCCACTGTGACAA | chr18 | 23078807 | 13586 | 1 | 0,0 | 56914 | 0 | 0,0 |
| OFT#9 | 124 | CGTGGGCCAGCCTGGGACAA | chrX | -153625648 | 14991 | 0 | 0,0 | 20784 | 0 | 0,0 |
| OFT#10 | 125 | AGAGAGCCACACTGAGACAG | chr5 | 133765919 | 40508 | 8 | 0,0 | 30937 | 0 | 0,0 |

### Targeting EWSR1-FLI1 fusion gene with CRISPR-Cas9 blocks tumour growth in vivo

To evaluate whether CRISPR fusion gene deletion can *in vivo* control human cancer growth in athymic mice we used an adenoviral delivery approach. Wild type A673 cells were subcutaneously injected in the flank of athymic mice (Day 0). The xenografted tumours were allowed to grow for two weeks (Day 10) until reached ∼150 mm³ in size. These tumours were then injected with 2.5 x 10⁹ plaque-forming units (pfu) of Ad/sgE3.2sgF8.2Cas9, Ad/Cas9 or PBS four times at days 10, 13, 16 and 19 (Figure 10a). Adenoviral delivery of sgRNAs and the Cas9 nuclease led to significant tumour growth inhibition, resulting in an average tumour size of 298.66 (+92.77) mm³ (P<0.05). Tumour volumes in control groups treated with either PBS or Ad/Cas9 increased over time and reached an average size of 1143.98 (+337.59) mm³ or 1345.25 (+685.16) mm³, respectively, at the end of the treatment (Figure 10b). Thus, the *EWSR1-FLI1* edited experimental cells reduced tumour size by 83.5 % compared to the controls, respectively. Immunohistochemic staining using Cas9 antibody confirmed expression of Cas9 protein in tumours injected with Ad/sgEWSR1-sgFLI1-Cas9 (Figure 10c). The antitumor efficacy of the fusion gene deletion approach was further investigated by histological and immunohistochemical analysis. H&E staining revealed a markedly reduced number of viable tumour cells and more extensive necrotic regions in CRISPR edited cells than in controls. Moreover, CRISPR edited cells exhibited 80 % lower levels of KI67 proliferation marker compared with control tumours (Figure 10d). Fusion gene deleted tumours showed a ≈30 % increase in caspase-3 protein expression compared with control tumours (Figure 11d). Importantly, mice treated with Ad/sgE3.2sgF8.2Cas9 showed no associated mortality in the 80 days of the study, whereas all controls needed to be sacrificed after two weeks of cell injection (Figure 10e). These results confirmed the therapeutic efficacy of the fusion gene edition approach to treat in vivo tumours driven by *EWSR1-FL11* fusion gene.

### Strategy for targeted of the chronic myeloid leukaemia tyrosine kinase BCR-ABL fusion gene in K562 cells

To evaluate whether such gene editing approach might be used as a universal approach for fusion gene driver cancer treatment, we reproduced a similar strategy to delete a classical tyrosine kinase fusion gene. The inventors choose *BCR-ABL1* generated by the t(9;22)(q34;q11) translocation, genetic abnormality hallmark of CML. *BCR-ABL1* creates a constitutively active tyrosine kinase, which leads to uncontrolled proliferation. We followed the same methodological approach described above. Briefly, four pairs of sgRNAs targeting *BCR* intron 8 and *ABL* intron 1 regions were designed (Figure 11a and Table 2). Following NHEJ these would result in deletion of 133.9kb of genomic *BCR-ABL1* DNA. Successful deletion will remove a large portion of the *BCR* Dbl homology (DH) domain, together with the frameshift alteration of the entire *ABL1* DNA binding domain. Four sgRNAs combinations were cloned to generate the pLV-U6BH1A-C9G (BA1, BA2, BA3 and BA4) vectors. Then, we examined the efficiency to generate *BCR-ABL1* targeted deletion in CML patient derived K562 cells that harbour the p210 isoform of the *BCR-ABL1* fusion gene. The efficient reduction of BCR-ABL mRNA was confirmed 24h post-nucleofection by RT-PCR and Sanger sequencing (Figure 11b). Colony forming assays on metilcellulose agar confirmed that targeted deletion of *BCR-ABL1* induced dramatic reduction on proliferation and death in K562 cells. Quantitative data analysis showed that the *BCR-ABL1* deletion significantly suppressed colony formation to approximately 85 % (Figure 11c). Quantitative data analysis of SubG1 analysis with the four combinations of sgRNAs in K562 experimental and control cells after 72 h in culture showed significant increase of apoptosis in the *BCR-ABL1* deleted cells compared to control cells (P<0.05). (Figure 11d).

To evaluate *BCR-ABL1* deletion effects in vivo, K562 cells were subcutaneously injected in the flank of athymic mice following the same strategic approach described above. After three weeks of growth the tumours were injected with 2.5 x 10⁹ plaque-forming units (pfu) of Ad/sgBA1-Cas9 or PBS four times at days 16, 19, 22 and 24. Adenoviral delivery of both targeting sgRNAs and the Cas9 nuclease led to significant tumour growth inhibition, resulting in an average tumour size of 128.77 (+63.53) mm³ (P<0.05). Tumour volumes in control groups treated with PBS increased over time and reached an average size of 1853.91 mm³, 6 days after treatment (Figure 12a and b). Importantly, mice treated with Ad/sgBA1Cas9 showed no associated mortality in the 60 days of the study, whereas all controls needed to be sacrificed after two weeks of cell injection.

### Robust CRISPR-Cas13-mediated knockdown of EWSR1-FLI1 and BCR-ABL1 mRNA expression in ES and CML cancer cell lines

To achieve the highest possible silencing of EWSR1-FLI1 and BCR-ABL1 mRNAs using the CRISPR-Cas13 system, the Cas13 protein and crRNAs are expressed from a lentiviral vector, LV-Cas13-crRNA. A series of crRNAs are tested to choose the most efficient (a representative example is shown in Table 5). The guide fragments in the series of crRNAs cover all of the positions containing the EWSR1-FLI1 and BCR-ABL1 breakpoints. The analysis of EWSR1-FLI1 or BCR-ABL1 mRNA expression levels of Cas 13-crRNA lentivirus transduced ES or CML cancer cells show a decrease after transduction. The crRNAs producing the highest EWSR1-FLI1 or BCR-ABL1 mRNA knockdown are chosen for subsequent experiments.

### Table 5. crRNAs

**> crRNA DNAJB1-PRKACA**
   SEQ ID NO: 144 GCTACGGGGAGGAAGTGAAAGAATTCTTAG
**> crRNA EML4-ALK**
   SEQ ID NO: 145 GGAAAGGACCTAAAGTGTACCGCCGGAAGC
**> crRNA PAX3-FOXO1**
   SEQ ID NO: 146 GGCAGTATGGACAAAAATTCAATTCGTCAT
**> crRNA TPM3-NTRK1**
   SEQ ID NO: 147 GATAAACTCAAGGAGACACTAACAGCACAT

### EWSR1-FLI1 and BCR-ABL1 knockdown by CRISPR-Cas13 is highly specific and blocks the proliferation of ES and CML cancer cell

The antitumor effects of CRISPR-Cas13-mediated EWSR1-FLI1 and BCR-ABL mRNA knockdown is evaluated in ES and CML cancer cells. First, we confirm that LV transduction of Cas13 and crRNA significantly reduces EWSR1-FLI1 and BCR-ABL mRNA expression levels in a time-dependent manner. Long-term and soft agar cell culture together with subG1 assay are used to measure the cell growth rate and apoptosis levels in cells treated with the CRISPRCas13 system. Transduction with the LV-Cas13-crRNA vector significantly suppress the growth and increase apoptotic cell rates of ES and CML cancer cells. There is no obvious change in the growth or apoptosis rates in control cells after treatment with the CRISPR-Cas13 system.

### KRAS-g12d knockdown with CRISPR-Cas13 inhibits tumour growth in vivo.

To explore the antitumor potency of the CRISPR-Cas13 system *in vivo,* mice bearing subcutaneous ES or CML xenografts are treated with repeated intratumoural injections of the optimized CRISPR-Cas13 system delivered by adenoviral vectors. Compared to the control group, the Cas13-crRNA treated tumours group shows a significant volume reduction. The antitumour efficacy of the EWSR1-FLI1 or BCR-ABL1 knockdown approach is further investigated by histological and immunohistochemical analysis. H&E staining reveals a markedly reduce number of viable tumour cells and more extensive necrotic regions in treated cells than in controls. Moreover, CRISPR edited cells exhibit lower levels of KI67 proliferation marker compared with control tumours. Fusion mRNA knockdown tumours show an increase in caspase-3 protein expression compared with control tumours. Importantly, mice treated with Ad/Cas13-crRNA show lower mortality during the study.

### Bibliography:

1. Rabbitts TH. Nature 372, 143-149 (1994). DOI: 10.1038/372143a0.
2. Mitelman F et al. Mitelman database of chromosome aberrations and gene fusions in cancer. NCI [online] (2017).
3. Mitelman F et al. Nat Rev Cancer 2007; 7(4):233-245.
4. Licht JD. N Engl J Med 2009; 360 (9):928-930.
5. Druker BJ et al. N Engl JMed 2001; 344 (14):1031-1037.
6. Borden EC et al. Clin. Cancer Res. 9, 1941-1956 (2003). PMID:12796356
7. Johansson B et al. Ann Med. 36, 492-503 (2004). DOI:10.1080/07853890410018808
8. Mrózek K et al. Blood Rev. 18, 115-36(2004).DOI:10.1016/S0268-960X(03)00040-7
9. Mitelman F et al. Gen Chrom & Cancer 43, 350-66 (2005). DOI:10.1002/gcc.20212
10. Antonescu CR. Histopathol 48, 13-21(2006). DOI:10.1111/j.1365-2559.2005.02285.x
11. Oehler VG & Radich JP. Curr Oncol Rep. 5, 426-435 (2003). PMID:12895396
12. Avigad S et al. Cancer 100, 1053-1058 (2004). DOI:10.1002/cncr.20059
13. Deininger M et al. Blood 105, 2640-2653 (2005). DOI:10.1182/blood-2004-08-3097
14. Kern W et al. Crit Rev Oncol Hematol. 56, 283-309 (2005). DOI:10.1016/j.critrevonc.2004.06.004
15. Rodriguez-Garcia A et al. Curr Cancer Drug Targets 1, 109-19(2001). PMID:12188884
16. Thomas et al. Act Pharmcol 27, 273-81 (2006) DOI:10.1111/j.1745-7254.2006.00282.x
17. Mojica et al. J Mol Evol 60, 174-182 (2005). DOI:10.1007/s00239-004-0046-3
18. Jinek et al. Science 337, 816-821 (2012). DOI:10.1126/science. 1225829
19. Esvelt et al. eLife 3, 03401 (2014). DOI:10.7554/eLife.03401
20. Chen et al. Nat Biotech 35 (6) 543-550 (2017). DOI:10.1038/nbt.3843
21. P. Mohanraju, et al. Science 353 (2016) aad5147.
22. O.O. Abudayyeh, et al. Science 353 (2016) aaf5573.
23. J.S. Gootenberg, et al. Science 356 (2017) 438-442.
24. L. Liu, et al. Cell 170 (2017) 714-726 e710.
25. L. Liu, et al. Cell 168 (2017) 121-134 e112.
26. A. East-Seletsky, et al. Mol. Cell 66 (2017) 373-383 e373.
27. A. East-Seletsky, et al. Nature 538 (2016) 270-273.
28. D.B.T. Cox, et al. Science 358 (2017) 1019-1027.
29. O.O. Abudayyeh, et al. Nature 550 (2017) 280-284.
30. X. Zhao et al. Cancer Letters 431 (2018) 171-181.

## Claims

1. A method for eliminating cancer cells, wherein said cells comprise a genomic rearrangement which leads either to the expression of a fusion gene not present in non-cancer cells, or to genomic amplifications or rearrangements which lead to the induction of the expression or to the overexpression of a cancer inducing gene, said method comprising:
a. cleaving the genome in at least two sites, said cleavage leading to either a deletion, an inversion, a frameshift and/or an insertion in the genome of said cancer cells, and/or
b. cleaving the expression product of said fusion gene or cancer inducing gene in at least one site.

2. The method according to the preceding claim, wherein the cancer cells comprise a genomic rearrangement which leads to the expression of the rearranged gene not present in non-cancer cells, preferably leads to the expression of a fusion gene not present in non-cancer cells.

3. The method according to any one of the preceding claims, wherein said method comprises cleaving the genome in two sites, or in three sites or in four sites.

4. The method according to any one of the preceding claims, wherein cleaving the genome leads to a deletion, an inversion, a frameshift or any combination thereof.

5. The method according to any one of the preceding claims, wherein at least two of the cleaving sites are in introns.

6. The method according to any one of the preceding claims, wherein the cleaving is done by an endonuclease selected from a CRISPR associated protein, a zinc-finger nuclease (ZFN) and a transcription activator-like effector nuclease (TALEN).

7. The method according to any one of the preceding claims, wherein the cleaving is done by a Cas protein, preferably Cas9 or Cas13, more preferably Cas9.

8. The method according to any one of the two preceding claims, wherein at least one guide RNA (gRNA) is used to target the cleaving of the genome, preferably at least two gRNAs are used to target the cleaving of the genome.

9. The method according to any one of the two preceding claims, wherein the target of said endonuclease is in an exon or intron of a fusion gene present in cancer cells and absent in non-cancer cells and wherein said target is not patient-specific.

10. A kit of parts comprising at least two endonucleases, preferably selected from a zinc-finger nuclease (ZFN) and a transcription activator-like effector nuclease (TALEN), wherein said endonucleases specifically cleave the genome in at least two sites and wherein said cleavage leads to either a deletion, a frameshift and/or an insertion in the genome, preferably a deletion and/or a frameshift.

11. A kit of parts comprising:
a. a CRISPR associated endonuclease, preferably a Cas protein, more preferably Cas9 or Cas13, most preferably a Cas9;
b. at least one gRNA that has a targeting domain in a genomic rearrangement present in a cancer cell which leads to genomic amplifications which lead to the induction of the overexpression of a cancer inducing gene; or at least two gRNAs that have a targeting domain in a genomic rearrangement present in a cancer cell which leads either to the expression a fusion gene not present in non-cancer cells or to rearrangements which lead to the induction of the expression or to the overexpression of a cancer inducing gene.

12. The kit of parts according to the preceding claims comprising:
a. the nuclease with amino acid sequence SEQ ID NO: 1; and
b. the pair of gRNAs with nucleotide sequences SEQ ID NO: 2 and SEQ ID NO: 3; or the pair of gRNAs with nucleotide sequences SEQ ID NO: 4 and SEQ ID NO: 5; or a pair of gRNAs with nucleotide sequences SEQ ID NO: 128 or SEQ ID NO: 129 and SEQ ID NO: 130 or SEQ ID NO: 131; or a pair of gRNAs with nucleotide sequences SEQ ID NO: 132 or SEQ ID NO: 133 and SEQ ID NO: 134 or SEQ ID NO: 135; or a pair of gRNAs with nucleotide sequences SEQ ID NO: 136 or SEQ ID NO: 137 and SEQ ID NO: 138 or SEQ ID NO: 139; or a pair of gRNAs with nucleotide sequences SEQ ID NO: 140 or SEQ ID NO: 141 and SEQ ID NO: 142 or SEQ ID NO: 143.

13. A nucleic acid comprising the codifying sequence for:
a CRISPR associated endonuclease, preferably a Cas protein, more preferably Cas9 or Cas13, even more preferably Cas9;
b. at least one gRNA that has a targeting domain in the expression product of a fusion gene or at least a pair of gRNAs that have a targeting domain in a genomic rearrangement present in a cancer cell which leads either to the expression of a fusion gene not present in non-cancer cells, or to genomic amplifications or rearrangements which lead to the induction of the expression or to the overexpression of a cancer inducing gene.

14. A nucleic acid comprising the codifying sequence for:
a. the nuclease with amino acid sequence SEQ ID NO: 1; and
b. the pair of gRNAs with nucleotide sequences SEQ ID NO: 2 and SEQ ID NO: 3; or the pair of gRNAs with nucleotide sequences SEQ ID NO: 4 and SEQ ID NO: 5; or a pair of gRNAs with nucleotide sequences SEQ ID NO: 128 or SEQ ID NO: 129 and SEQ ID NO: 130 or SEQ ID NO: 131; or a pair of gRNAs with nucleotide sequences SEQ ID NO: 132 or SEQ ID NO: 133 and SEQ ID NO: 134 or SEQ ID NO: 135; or a pair of gRNAs with nucleotide sequences SEQ ID NO: 136 or SEQ ID NO: 137 and SEQ ID NO: 138 or SEQ ID NO: 139; or a pair of gRNAs with nucleotide sequences SEQ ID NO: 140 or SEQ ID NO: 141 and SEQ ID NO: 142 or SEQ ID NO: 143.

15. Use of the kit of parts of claims 10 or claim 11 or claim 12 or the nucleic acid of claim 13 or claim 14 for the treatment of cancer, preferably for the treatment of fibrolamellar hepatocellular carcinoma, non-small cell lung cancer, alveolar rhabdomyosarcoma, glioblastoma, colorectal cancer, acute lymphocytic leukemia, Ewing sarcoma and bladder cancer.
